# EUROPEAN PATENT APPLICATION

(11) **EP 4 360 658 A1**
(43) Date of publication of application: **01.05.2024**
(21) Application number: 22204457.0
(22) Date of filing: 28.10.2022
(51) Int. Cl.: A61K 49/00, C07K 7/06, C07K 7/08

(54) **COMPOUNDS FOR DETECTION OF ORAL INFLAMMATION**

(71) Applicant: Julius-Maximilians-Universität Würzburg, 97070 Würzburg (DE)
(72) Inventor: MEINEL, Lorenz, 97082 Wuerzburg (DE); TER MORS, Björn, 49076 Osnabrück (DE); DRIESSEN, Marc, 47839 Krefeld (DE)
(74) Representative: Kraus & Lederer PartGmbB

(57) **Abstract**

The present invention relates to novel protease-sensitive peptides, related compounds or salts thereof having the structure Den-R-NH-Pep (wherein Den is a denatonium residue and Pep designates a protease-sensitive peptide) as well as bioresponsive sensors comprising same, which may be used for diagnostic purposes, in particular for detection of inflammation, as well as to related methods and uses.

## Description

### FIELD OF THE INVENTION

The present invention essentially relates to the field of diagnostic peptides, especially peptides that are proteolytically cleavable by enzymes, as well as to respective compounds, salts, sensors, and particular uses thereof in medicine, particularly for detecting a medical condition, especially for detecting (oral) inflammation.

### BACKGROUND

Inflammations in the oral cavity often occur in the form of swelling and redness, and can be extremely painful. Sometimes it comes to bleeding of the inflamed area. However, especially at an early stage, these inflammations often remain unrecognized by the patients or the severe consequences, if not treated, are underestimated.

Chronic inflammations of the periodontium, such as gingivitis and periodontitis, are among the most common oral diseases worldwide. If left untreated, they can lead to tooth loss. This is accompanied by significant functional limitations and considerable impairment of aesthetics and phonetics, leading to exclusion and impairment of participation in social life [1, 2]. Worldwide, periodontitis affects an estimated 20-50 % of the global population and around 40 % of the adult population in the US [3, 4]. The consequential costs for the healthcare system of a late or undetected disease are high [5, 6].

Periodontitis is a chronic inflammatory disease closely associated with a dysbiotic, pro-inflammatory change in the composition of the oral microbiota, progressively destroying the integrity of the periodontium with subsequent tooth mobility and tooth loss as clinical outcome. Early diagnosis and early treatment leverage overall clinical success and may lead to preservation. Therefore, periodontal inflammation should be detected as soon as possible.

In other words, periodontitis may be described as a chronic inflammatory process fueled by microbial dysbiosis, dental plaque formation, genetic predisposition, systemic diseases, smoking, or lifestyle [7]. Progressing inflammation drives irreversible damage to the tooth-supporting tissues and, ultimately, tooth loss [8].

Therefore, therapy aims to start in the early stages of the disease [9]. The sooner inflammations in the oral cavity are detected and treated, the greater the chance of preventing the occurrence of irreversible damage and maintaining oral health into old age. However, detection finds limits such as frequency of check-ups or patient adherence to regular visits. Ideally, highly intuitive diagnostic tools would be available, which anyone could use anywhere and anytime for personal check-ups. Besides, self-diagnosis is generally not possible. The clinical dental examination includes measuring pocket probing depth, recession, bleeding on probing (BoP), tooth mobility, furcation, and radiographic findings of bone loss [9].

Moreover, unfortunately, periodontitis is initially often symptom-free and not noticed or noticed at an advanced state by affected patients. Thus patient compliance, even where patients are entitled to use the offer of an annual dental check-up of their oral health, is often insufficient, and an early diagnosis can usually not be guaranteed [9, 10].

Furthermore, in the prior art, WO 2013/131993 already discloses a means for the diagnosis of inflammatory tissues in dental application. Besides, US Pat. No. 9,526,803 provides an approach for the direct detection of pathogens. In both of said documents, a poly(methylmethacrylate) (PMMA) substrate and/or anchor for attachment of the flavoring or colorant substance via a linker is used.

In addition, Ritzer J et al report on sensory chewing gums as a bioresponsive system for the detection of periodontal disease and peri-implantitis [13]. In said reference, denatonium is used, which is the bitterest compound currently known. Due to its extremely bitter taste, it is used in cosmetics and household products as an additive to prevent inadvertent ingestion. Particular denatonium derivatives are disclosed in previous application WO 2019/162289 - and particular for the gustatory detection of inflammation in the oral cavity are already disclosed in previous application WO 2020187750.

In any case, it is generally (still) desired to find a reliable, specific, uncomplicated and rapid way of diagnosing such diseases at the earliest possible stage, preferably by the patient himself without having to consult a dentist. Particularly preferable solutions include easy to use products for daily use. Moreover, there remains a need of improving the available diagnostic tools to facilitate detection.

### SUMMARY OF THE INVENTION

The present invention addresses all of the above objects and needs by providing respective solutions as defined in the claims.

Generally, the present invention provides subject-matter as defined in the present claims.

In summary, the invention particularly relates to the subject matter defined in the following items [1] to [66]:
[1] A compound having the structure Den-R-NH-Pep, or a salt thereof, wherein Den is a detectable label, preferably an optionally substituted denatonium residue,
   R is an optionally substituted C₁₋₃ alkylene group, particularly wherein R is CH₂,
   Pep is a protease-sensitive peptide,
   wherein the C-terminus of said peptide forms an amide bond with the group -NH-,
   and wherein said protease-sensitive peptide Pep comprises amino acid residues having the sequence P4-P3-P2-P1-P1'-P2'-P3'-P4', wherein P4 is G,
   P3 is independently selected from A and P,
   P2 is independently selected from L and Q,
   P1 is independently selected from G, A and E, preferably from A and E,
   P1' is independently selected from I, L and Y,
   P2' is independently selected from V and W,
   P3' is G, and
   P4' is A.
[2] The compound or salt of item [1], having the formula [I]: 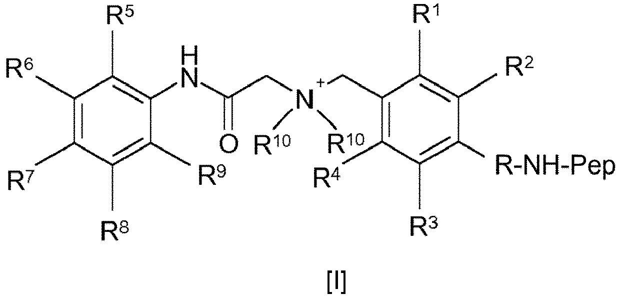 wherein
   R¹⁰ is hydrogen or C₁-C₁₀-alkyl,
   R¹-R⁹ independently are hydrogen, halogen, C₁-C₅-alkyl, C₁-C₄-alkoxy, C₁-C₂₀-alkoxycarbonyl,
   R and Pep are as defined in item [1].
[3] The compound or salt of any of items [1] to [2], wherein
   R¹⁰, R⁵ and R⁹ is C₁-C₄-alkyl,
   R¹-R⁴ and R⁶-R⁸ is hydrogen,
   R and Pep are as defined in item [1].
[4] The compound or salt of any of items [1] to [3], wherein
   R¹⁰ is ethyl,
   R⁵ and R⁹ is methyl,
   R¹-R⁴ and R⁶-R⁸ is hydrogen,
   R and Pep are as defined in item [1].
[5] The compound or salt of item [1],
   wherein the compound has the formula [Ia]: 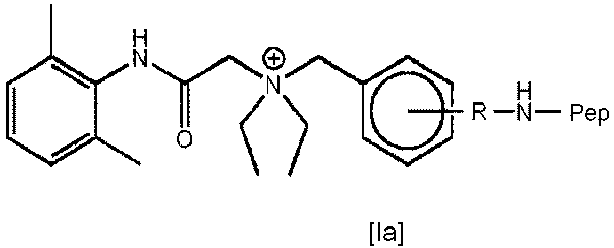 and wherein R and Pep are as defined in item [1].
[6] The compound or salt of any of items [1] to [5],
   wherein the compound has the formula [Ib]: 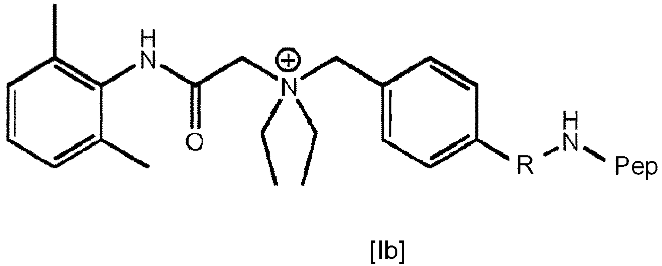 and wherein R and Pep are as defined in item [1].
[7] The compound or salt of any of items [1] to [6], wherein the protease-sensitive peptide comprises at least 8 amino acids, preferably 8 to 24 amino acids, more preferably 8 to 16 amino acids.
[8] The compound or salt of any of items [1] to [7], wherein the protease-sensitive peptide consists of 8 amino acids, preferably of 8 to 24 amino acids, more preferably of 8 to 16 amino acids.
[9] The compound or salt of any of items [1] to [8],
   wherein said protease-sensitive peptide Pep comprises amino acid residues having the sequence P4-P3-P2-P1-P1'-P2'-P3'-P4', wherein
   P4 is G,
   P3 is independently selected from A and P,
   P2 is L,
   P1 is independently selected from A and E,
   P1' is independently selected from I and L,
   P2' is V,
   P3' is G, and
   P4' is A;
   or wherein said protease-sensitive peptide Pep comprises amino acid residues having a sequence selected from GPQAYWGA (SEQ ID NO: 10) and GPLAYWGA (SEQ ID NO: 11).
[10] The compound or salt of any of items [1] to [9],
   wherein said protease-sensitive peptide Pep comprises amino acid residues having a sequence selected from the group consisting of
   GPLELVGA (SEQ ID NO: 2),
   GPLEIVGA (SEQ ID NO: 3),
   GPLALVGA (SEQ ID NO: 4),
   GPLAIVGA (SEQ ID NO: 5),
   GALELVGA (SEQ ID NO: 6),
   GALEIVGA (SEQ ID NO: 7),
   GALALVGA (SEQ ID NO: 8),
   GALAIVGA (SEQ ID NO: 9),
   GPQAYWGA (SEQ ID NO: 10) and
   GPLAYWGA (SEQ ID NO: 11).
[11] The compound or salt of any of items [1] to [10],
   wherein said protease-sensitive peptide Pep comprises amino acid residues having a sequence selected from the group consisting of GPLELVGA (SEQ ID NO: 2), GPLEIVGA (SEQ ID NO: 3), GPLALVGA (SEQ ID NO: 4), GPLAIVGA (SEQ ID NO: 5), and GALALVGA (SEQ ID NO: 8), and GPQAYWGA (SEQ ID NO: 10).
[12] The compound or salt of any of items [1] to [9],
   wherein said protease-sensitive peptide Pep comprises amino acid residues having the sequence P4-P3-P2-P1-P1'-P2'-P3'-P4', wherein
   P4 is G,
   P3 is independently selected from A and P,
   P2 is L,
   P1 is independently selected from A and E,
   P1' is independently selected from I and L,
   P2' is V,
   P3' is G, and
   P4' is A.
[13] The compound or salt of any of items [1] to [9] and [12],
   wherein said protease-sensitive peptide Pep comprises amino acid residues having a sequence selected from the group consisting of GPLELVGA (SEQ ID NO: 2), GPLEIVGA (SEQ ID NO: 3), GPLALVGA (SEQ ID NO: 4), GPLAIVGA (SEQ ID NO: 5), GALELVGA (SEQ ID NO: 6), GALEIVGA (SEQ ID NO: 7), GALALVGA (SEQ ID NO: 8), and GALAIVGA (SEQ ID NO: 9).
[14] The compound or salt of any of items [1] to [9], [12] and [13],
   wherein said protease-sensitive peptide Pep comprises amino acid residues having a sequence selected from the group consisting of GPLELVGA (SEQ ID NO: 2), GPLEIVGA (SEQ ID NO: 3), GPLALVGA (SEQ ID NO: 4), GPLAIVGA (SEQ ID NO: 5), and GALALVGA (SEQ ID NO: 8).
[15] The compound or salt of any of items [1] to [11],
   wherein said protease-sensitive peptide Pep comprises amino acid residues having a sequence selected from the group consisting of GPLAYWGA (SEQ ID NO: 11) and GPQAYWGA (SEQ ID NO: 10).
[16] The compound or salt of any of items [1] to [11] or [15],
   wherein said protease-sensitive peptide Pep comprises amino acid residues having a sequence GPQAYWGA (SEQ ID NO: 10).
[17] The compound or salt of any of items [1] to [16], wherein said protease-sensitive peptide Pep further comprises one or two linker sequences.
[18] The compound or salt of any of items [1] to [17], wherein said protease-sensitive peptide Pep further comprises two linker sequences, particularly wherein said two linker sequences are designated as L1 and L2.
[19] The compound or salt of any of items [17] to [18], wherein each of said linker sequences comprises at least 4 amino acids, preferably 4 to 15 amino acids,
   in particular wherein each of said linker sequences consists of 4-6, preferably of 4 amino acids,
   especially wherein each linker comprises a sequence comprising two glycine and two serine residues,
   in particular wherein each linker comprises the sequence GSGS (SEQ ID NO: 18).
[20] The compound or salt of any of items [17] to [19], wherein said protease-sensitive peptide Pep further comprises two linker sequences L1 and L2, which are positioned on the N- and C-terminal side, respectively, of the amino acid residues of the peptide having a sequence as defined in any of the above items.
[21] The compound or salt of any of items [1] to [20], wherein the protease-sensitive peptide Pep comprises a stabilizing N-terminal modification.
[22] The compound or salt of any of items [1] to [21], wherein the protease-sensitive peptide Pep is acetylated at its N-terminus.
[23] The compound or salt of any of items [1] to [22], wherein the protease is a pathogen-specific protease.
[24] The compound or salt of item [23], wherein the pathogen is a bacterial pathogen.
[25] The compound or salt of item [23], wherein the pathogen is a viral pathogen.
[26] The compound or salt of any of items [1] to [25], wherein the protease is an endopeptidase.
[27] The compound or salt of any of items [1] to [26], wherein the said protease is a matrix metalloproteinase (MM).
[28] The compound or salt of any of items [1] to [27], wherein the said protease is a matrix metalloproteinase selected from the group consisting of MM-1, MM-8, and MM-9.
[29] The compound or salt of any of items [1] to [28], wherein the protease is matrix metalloproteinase 8.
[30] The compound or salt of any of items [1] to [29], wherein the protease-sensitive peptide is not selected from the group consisting of QPW (SEQ ID NO: 16), DAPV (SEQ ID NO: 17), GPQGIAGA (SEQ ID NO: 14), and GPQGIAGQ (SEQ ID NO: 15).
[31] The compound or salt of any of items [1] to [30], wherein the protease-sensitive peptide is not selected from the group consisting of GPQGIAGA (SEQ ID NO: 14), and GPQGIAGQ (SEQ ID NO: 15).
[32] The compound or salt of any of items [1] to [31], wherein the compound is not susceptible to cleavage by an aminopeptidase.
[33] The compound or salt of any of items [1] to [32], wherein the compound is not susceptible to cleavage by an exopeptidase.
[34] The compound or salt of any of items [1] to [33],
   comprising a counter ion selected from the group consisting of TFA⁻, F⁻, Cl⁻, Br⁻, 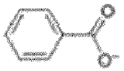 and 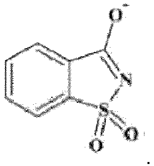
[35] The compound or salt of any of items [1] to [34],
   comprising a counter ion selected from the group consisting of F⁻, Cl⁻, Br⁻, 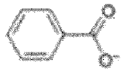 and 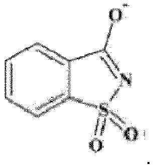
[36] The compound or salt according to any of the above items, for use in medicine.
[37] The compound or salt according to any of the above items, for use in a method of detecting a medical condition, particularly an inflammation.
[38] The compound or salt according to any of the above items, for use in a method of detecting an inflammation in the oral cavity of a human patient.
[39] The compound or salt for use of any of items [36] to [38], wherein the inflammation is detected by gustatory perception (by the patient).
[40] The compound or salt for use of any of items [36] to [39], wherein the inflammation is detected upon cleavage of the compound, thereby releasing a denatonium compound.
[41] The compound or salt for use of any of items [36] to [40], wherein, upon cleavage of the compound, a compound having the formula [IIa]: 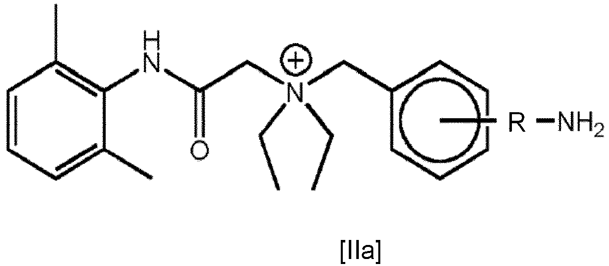 is released,
   wherein R is an optionally substituted C_{1 3} alkylene group, preferably CH₂.
[42] The compound or salt for use of any of items [36] to [41], wherein, upon cleavage of the compound, a compound having the formula [IIb]: 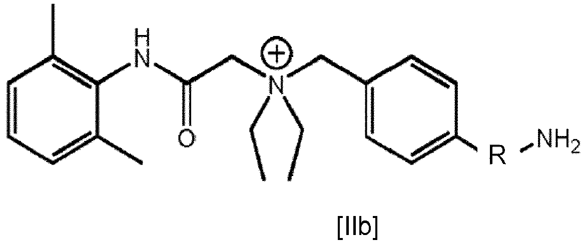 is released,
   wherein R is an optionally substituted C_{1 3} alkylene group, preferably CH₂.
[43] A bioresponsive sensor comprising the compound or salt of any one of items [1] to [35].
[44] The bioresponsive sensor of item [43], for use in medicine.
[45] The bioresponsive sensor according to item [43], for use in a method of detecting a medical condition, particularly an inflammation.
[46] The bioresponsive sensor according to item [43], for use in a method of detecting an inflammation in the oral cavity of a human patient.
[47] The bioresponsive sensor for use of any one of items [44] to [46], wherein the inflammation is detected by gustatory perception (by the patient).
[48] The bioresponsive sensor for use of any one of items [44] to [47], wherein the inflammation is detected upon cleavage of the compound, thereby releasing a denatonium compound.
[49] The bioresponsive sensor for use of any one of items [44] to [48], wherein, upon cleavage of the compound, a compound having the formula [IIa]: 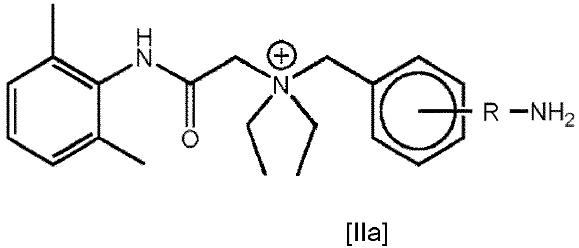 is released,
   wherein R is an optionally substituted C_{1 3} alkylene group, preferably CH₂.
[50] The bioresponsive sensor for use of any one of items [44] to [49], wherein, upon cleavage of the compound, a compound having the formula [IIb]: 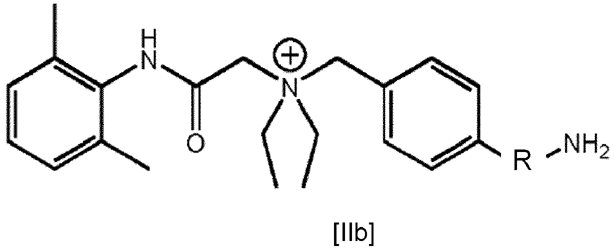 is released,
   wherein R is an optionally substituted C_{1 3} alkylene group, preferably CH₂.
[51] A method for the preparation of the compound of any one of items [1] to [35] or of the bioresponsive sensor of item [43], comprising the step of linking an amino-modified denatonium compound to the C-terminus of a protease-sensitive peptide.
[52] The method of item [51],
   wherein the amino-modified denatonium compound is a compound of formula [IIa] 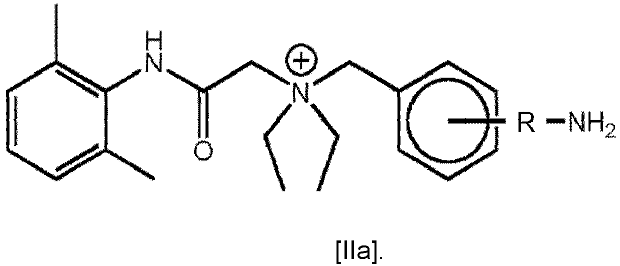
[53] The method of item [51] or [52],
   wherein the amino-modified denatonium compound is a compound of formula [IIb] 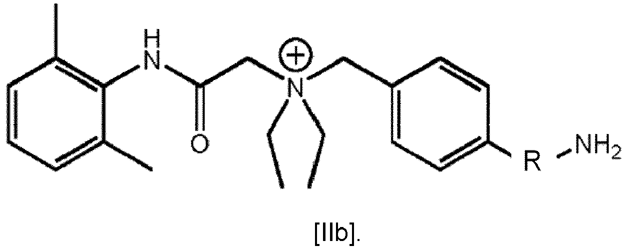
[54] The method of any one of items [51] to [53], further comprising, prior to the step of linking:
   - preparing a protease-sensitive peptide by solid phase peptide synthesis.
[55] The method of any one of items [51] to [54], further comprising, prior to the step of linking:
   - protecting the N-terminus of the protease-sensitive peptide with a protecting group, preferably acetic anhydride.
[56] A diagnostic chewing gum or a diagnostic confectionary, comprising the compound or salt of any one of items [1] to [35].
[57] A diagnostic chewing gum or a diagnostic confectionary, comprising the bioresponsive sensor of item [43].
[58] The diagnostic chewing gum of any one of items [56] to [57], for use in medicine.
[59] The diagnostic chewing gum of any one of items [56] to [57], for use in a method of detecting a medical condition, particularly an inflammation.
[60] The diagnostic chewing gum for use of item [59], wherein said method is further defined as in any one of items [36] to [42].
[61] A protease-sensitive peptide having a length of from 8 to 16 amino acids,
   wherein said protease-sensitive peptide Pep comprises amino acid residues having the sequence P4-P3-P2-P1-P1'-P2'-P3'-P4', wherein P4 is G, P3 is independently selected from A and P, P2 is independently selected from L and Q, P1 is independently selected from G, A and E, preferably from A and E, P1' is independently selected from I, L and Y, P2' is independently selected from V and W, P3' is G, and P4' is A.
[62] The protease-sensitive peptide of item [60], having a length of from 8 to 12 amino acids, preferably of 8 amino acids.
[63] The protease-sensitive peptide of any one of items [61] to [62],
   wherein said protease-sensitive peptide Pep comprises amino acid residues having the sequence P4-P3-P2-P1-P1'-P2'-P3'-P4', wherein P4 is G, P3 is independently selected from A and P, P2 is L, P1 is independently selected from A and E, P1' is independently selected from I and L, P2' is V, P3' is G, and P4' is A;
   or wherein said protease-sensitive peptide Pep comprises amino acid residues having a sequence selected from GPQAYWGA (SEQ ID NO: 10) and GPLAYWGA (SEQ ID NO: 11).
[64] The protease-sensitive peptide of any one of items [61] to [63], wherein said protease-sensitive peptide Pep comprises amino acid residues having a sequence selected from the group consisting of GPLELVGA (SEQ ID NO: 2), GPLEIVGA (SEQ ID NO: 3), GPLALVGA (SEQ ID NO: 4), GPLAIVGA (SEQ ID NO: 5), GALELVGA (SEQ ID NO: 6), GALEIVGA (SEQ ID NO: 7), GALALVGA (SEQ ID NO: 8), GALAIVGA (SEQ ID NO: 9), GPQAYWGA (SEQ ID NO: 10) and GPLAYWGA (SEQ ID NO: 11).
[65] The protease-sensitive peptide of any one of items [61] to [64], further defined as in any of the above items.
[66] The protease-sensitive peptide of any one of items [61] to [65] for use in medicine,
   particularly wherein the use is further defined as in any of the above items.
[67] The protease-sensitive peptide of any one of items [61] to [65] for use in a method of detecting a medical condition, particularly inflammation,
   particularly wherein the use is further defined as in any of the above items.

### DESCRIPTION OF THE FIGURES

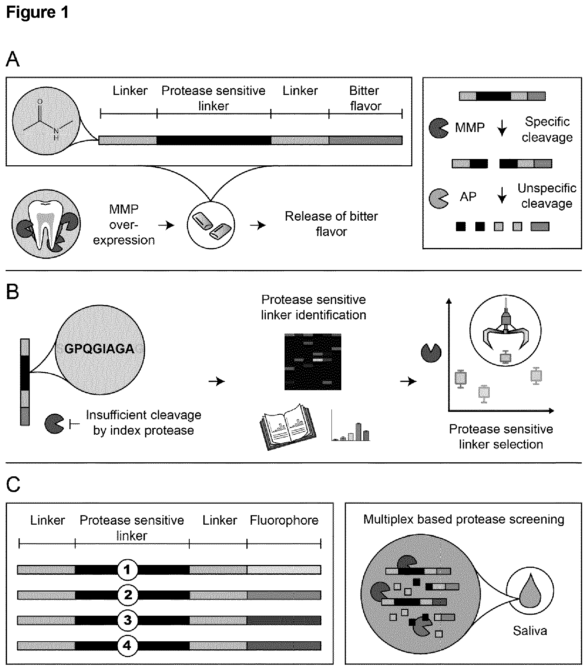

Figure 1: (A) The design and principle of the sensors. The index protease (MMP) specifically cleaves the protease-sensitive linker (PSL), resulting in fragments. These fragments are further digested by unspecific aminopeptidase (AP), leading to the release of a bitter substance. Acetylation of the sensor protects unspecific AP cleavage. (B) Cartoon of the steps for PSL identification and iterative improvement. (C) Final testing of selected PSLs in saliva using multiplexing

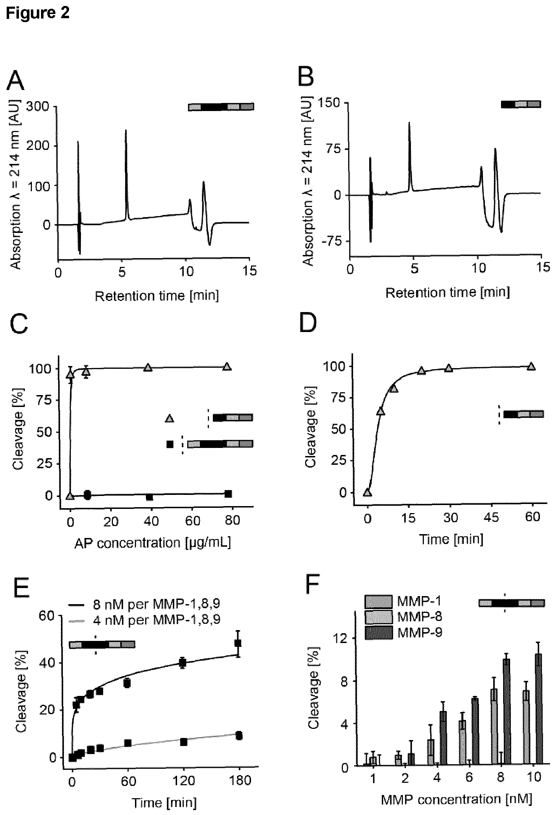

Figure 2: (A) High performance liquid chromatography (HPLC) chromatogram of the sensor (S1). (B) HPLC chromatogram of the fragment having the bitter substance of the sensor (S1c) after MMP cleavage. (C) Aminopeptidase (AP) cleavage of S1 and S1c at different AP concentrations. (D) AP cleavage of S1c over time. (E) MMP cleavage of S1 over time with a mixture of MMP-1, 8, 9. (F) S1 cleavage following exposure to MMP-1, 8, or 9.

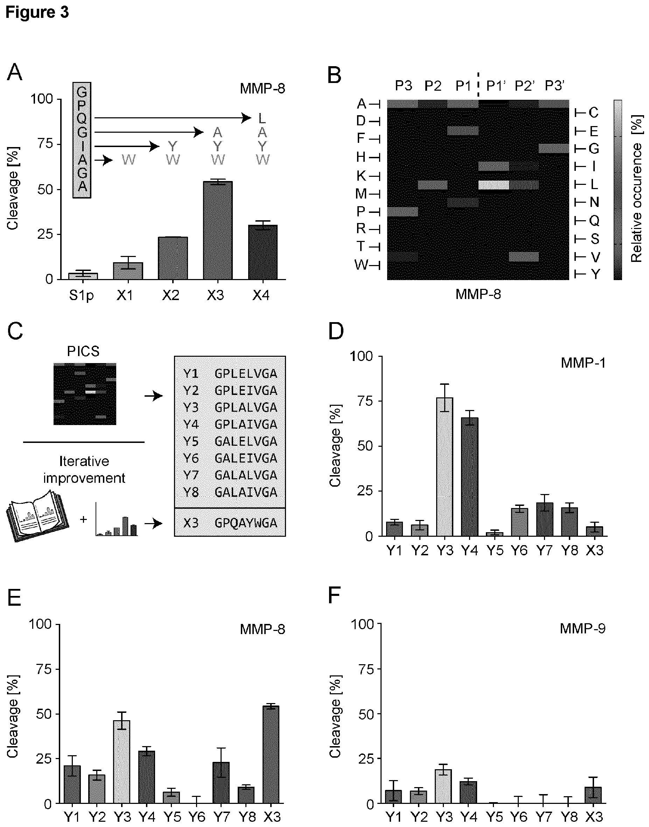

Figure 3: (A) Cleavage of protease-sensitive linkers (PSL) derived from S1 (GPQGIAGA). (B) PICS derived relative occurrence of amino acids for position P3 to P3' using the E. coli proteome. (C) The X series of new sensors is derived from optimization of S1 based on known relations of primary sequence and MMP-8 sensitivity (as in panel A). The y series is derived from the proteome searches (as in panel B). (D) MMP-1 cleavage, (E) MMP-8 cleavage, and (F) MMP-9 cleavage for selected PSLs.

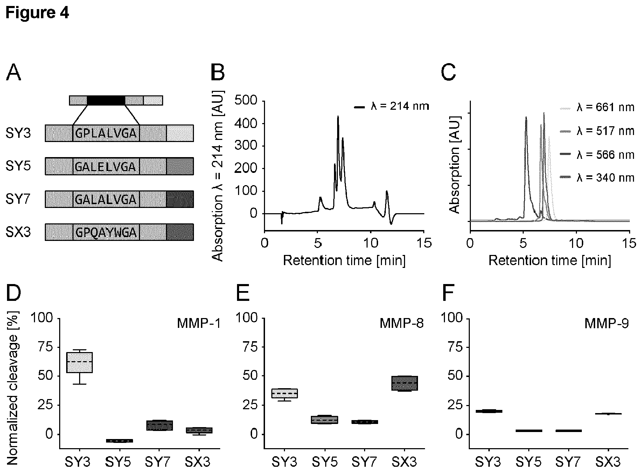

Figure 4: (A) Sensor overview. (B) HPLC chromatogram of mixture of SY3, SY5, SY7, and SX3. (C) HPLC chromatogram acquired at individual wavelengths for a mixture of SY3, SY5, SY7, and SX3. (D) Normalized MMP-1 cleavage, (E) MMP-8 cleavage and (F) MMP-9 cleavage for SY3, SY5, SY7, and SX3. Box plots show the mean as dotted line, whiskers indicate outliers and the range indicates the 25% and 75% percentiles, respectively.

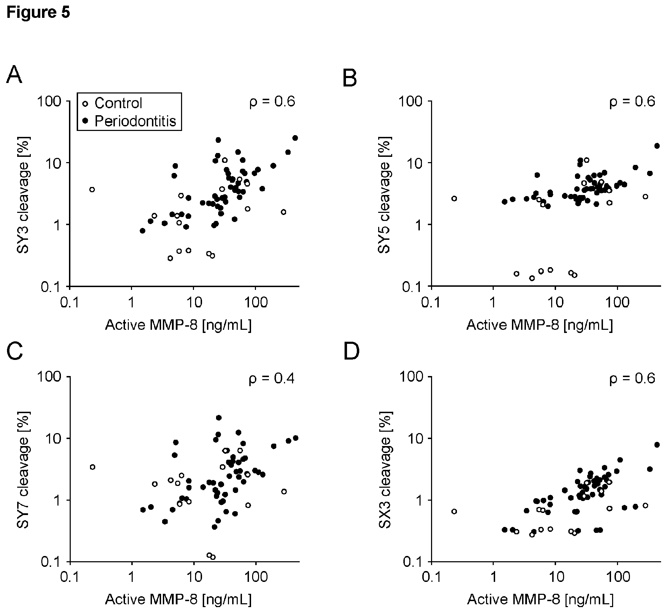

Figure 5: Cleavage of individual sensors in human saliva. Open circles result from the control group and filled circles from the periodontitis group. (A) SY3 cleavage (p=0.6), (B) SY5 cleavage (p=0.6), and (C) SY7 cleavage (ρ=0.4), and (D) SX3 cleavage (p=0.6) in response to the measured active MMP-8 concentration in saliva

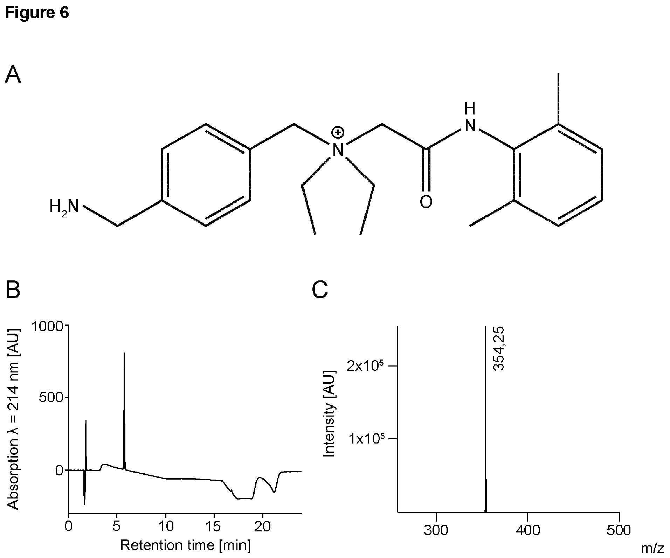

Figure 6: Characterization of denatonium derivative Den-CH2-NH2. (A) Structure of Den-CH2-NH2. (B) HPLC chromatogram of Den-CH2-NH2 indicating a purity of >95 %. (B) LC-MS analysis of Den-CH2-NH2 showing a m/z of 354.25 for a calculated m/z of 354.25.

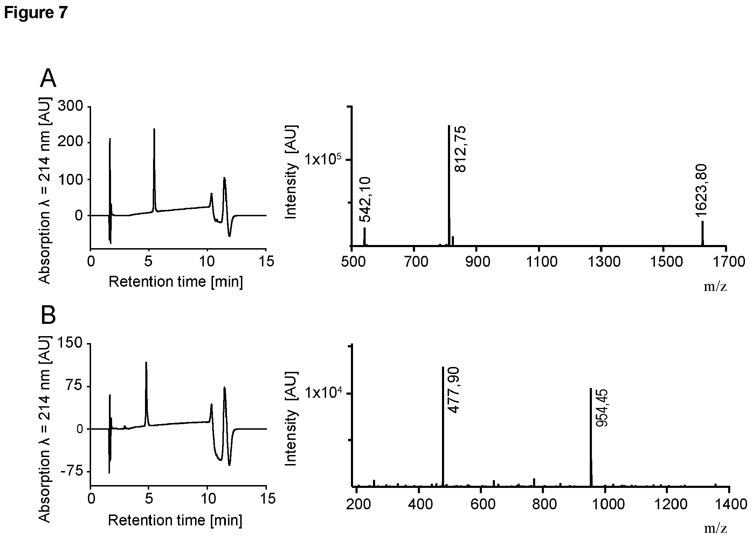

Figure 7 shows a characterization of S1 and S1c. (A) HPLC chromatogram of S1 indicating a purity of >95 %. LC-MS analysis of S1 indicating an observed m/z of 1623.80 for a calculated m/z of 1624.13. (B) HPLC chromatogram of S1c indicating a purity of >95 %. LC-MS analysis of S1c indicating an observed m/z of 954.45 for a calculated m/z of 954.54

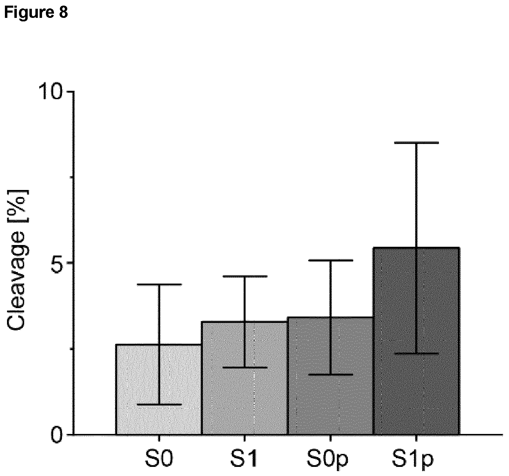

Figure 8 shows cleavage for different sensor stages. Cleavage results for S0, S1, S0p and S1p with 17.5 nM MMP-8 for 15 min at 37 °C. Experiments were performed with n = 3.

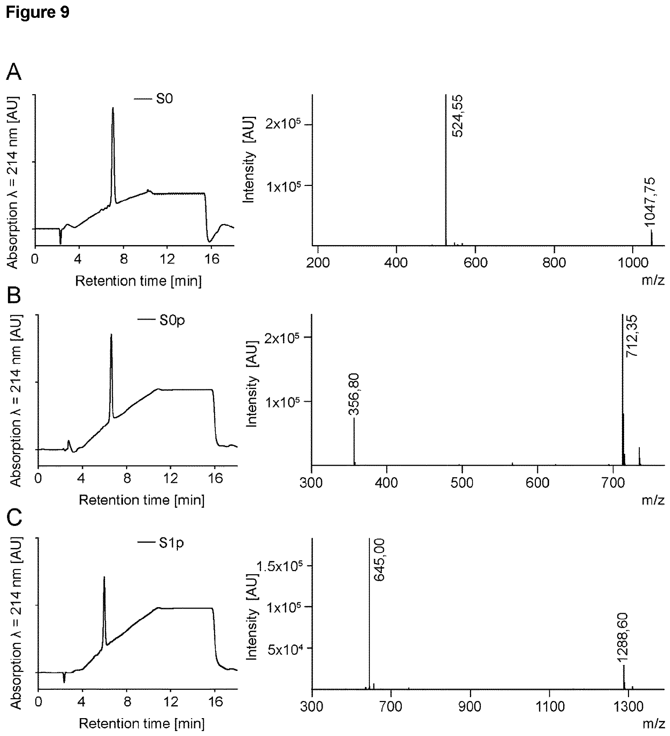

Figure 9 shows a characterization of S0, S1p and S0p. (A) LC-MS analysis of S0 indicating a purity of >95% and an observed m/z of 1047.75 for a calculated m/z of 1047.60. (B) LC-MS analysis of S0p indicating a purity of >95% and an observed m/z of 712.35 for a calculated m/z of 712.34. (C) LC-MS analysis of S1p indicating a purity of >95% and an observed m/z of 1288.60 for a calculated m/z of 1288.56

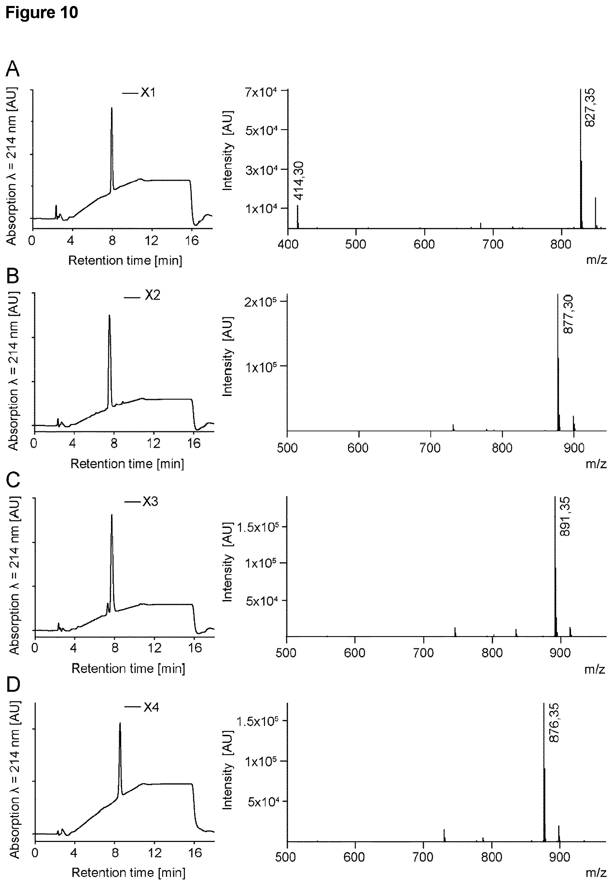

Figure 10 shows a characterization of X1-4. (A) LC-MS analysis of X1 indicating a purity of >95% and an observed m/z of 827.35 for a calculated m/z of 827.32. (B) LC-MS analysis of X2 indicating a purity of >95% and an observed m/z of 877.30 for a calculated m/z of 877.37. (C) LC-MS analysis of X3 indicating a purity of >90% and an observed m/z of 891.35 for a calculated m/z of 891.38. (D) LC-MS analysis of X4 indicating a purity of >95% and an observed m/z of 876.35 for a calculated m/z of 876.41.

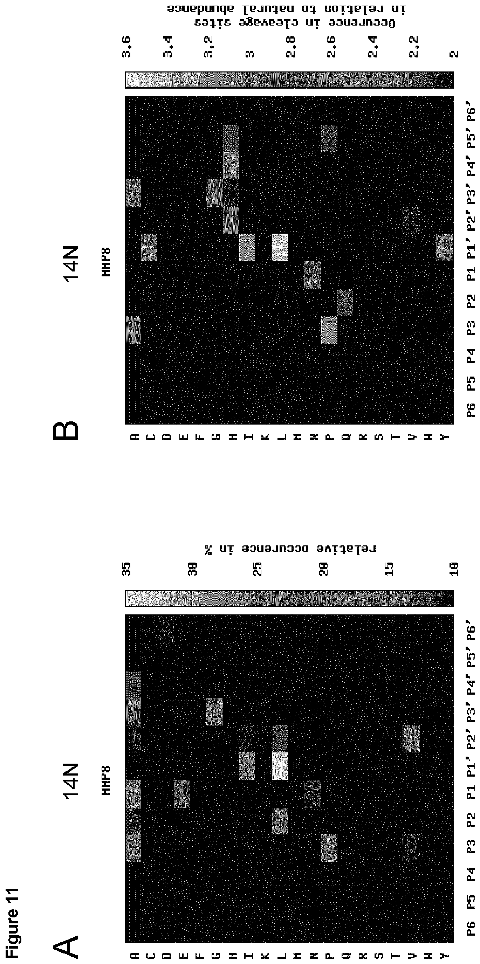

Figure 11 shows MMP-8 PICS results acquired with 14N E. coli proteome. (A) relative occurrence in %. (B) Occurrence in cleavage sites in relation to natural abundance.

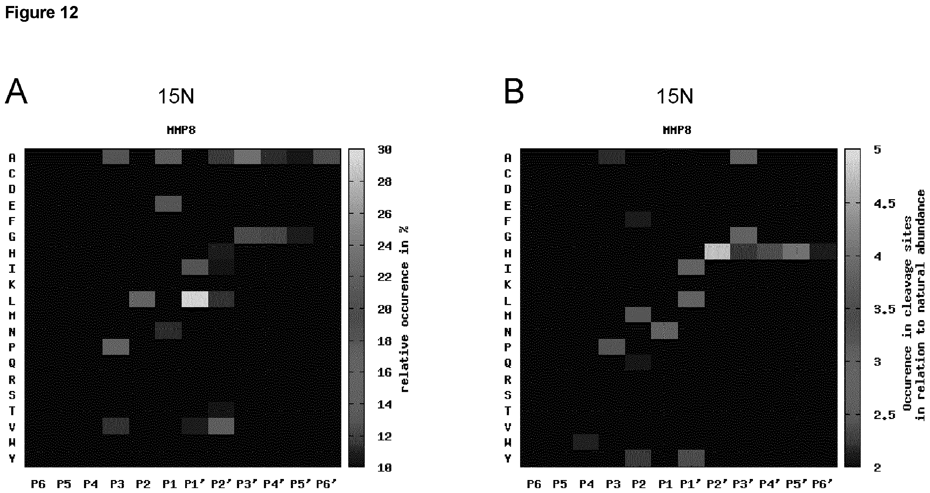

Figure 12 shows MMP-8 PICS results acquired with 15N E. coli proteome. (A) Relative occurrence in %. (B) Occurrence in cleavage sites in relation to natural abundance.

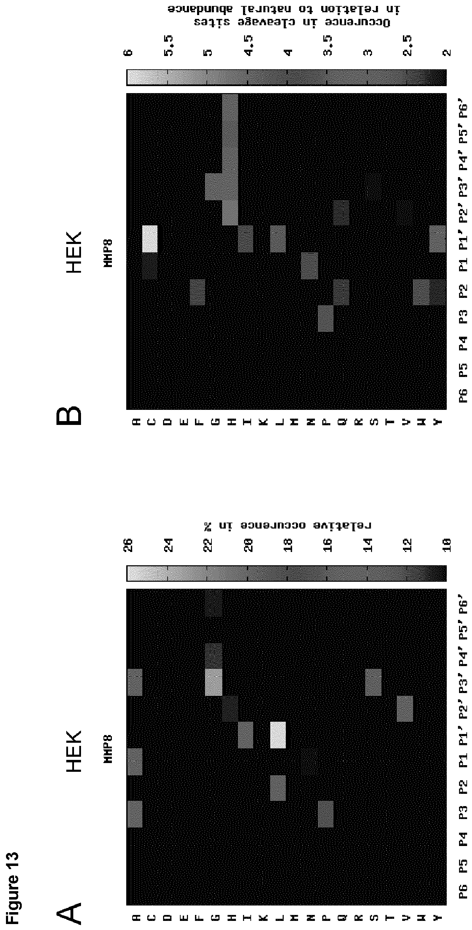

Figure 13 shows MMP-8 PICS results acquired with HEK proteome. (A) Relative occurrence in %. (B) Occurrence in cleavage sites in relation to natural abundance.

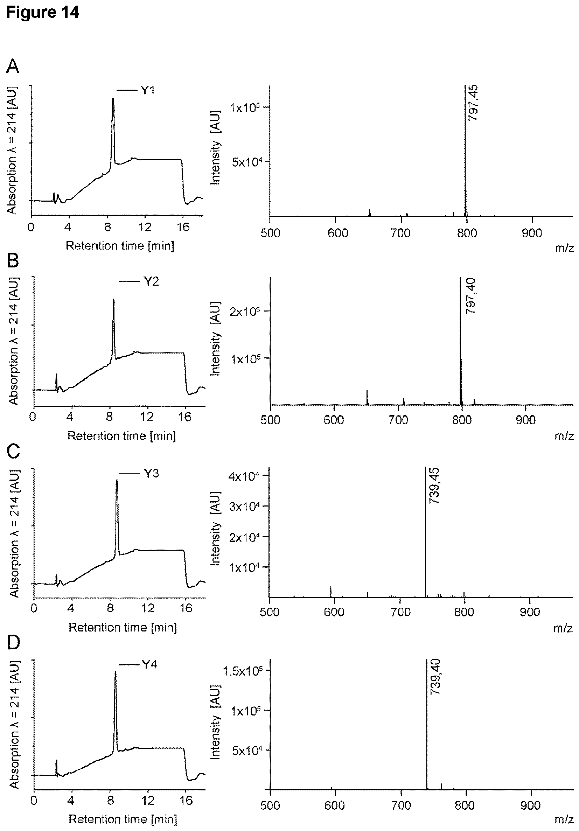

Figure 14 relates to the characterization of Y1-4. (A) LC-MS analysis of Y1 indicating a purity of >95% and an observed m/z of 797.45 for a calculated m/z of 797.42. (B) LC-MS analysis of Y2 indicating a purity of >95% and an observed m/z of 797.40 for a calculated m/z of 797.42. (C) LC-MS analysis of Y3 indicating a purity of >90% and an observed m/z of 739.45 for a calculated m/z of 739.42. (D) LC-MS analysis of Y4 indicating a purity of >95% and an observed m/z of 739.40 for a calculated m/z of 739.42.

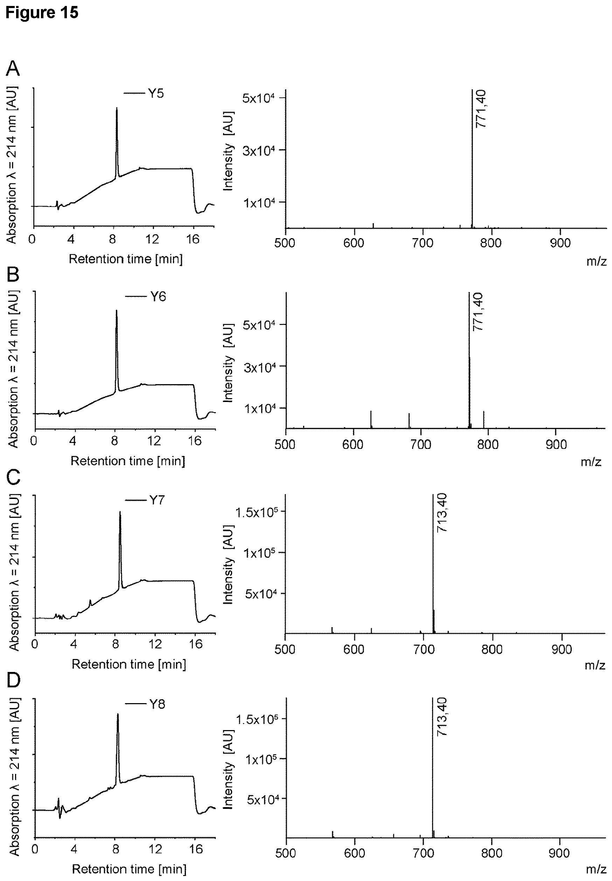

Figure 15 depicts a characterization of Y5-8. (A) LC-MS analysis of Y5 indicating a purity of >95% and an observed m/z of 771.40 for a calculated m/z of 771.41. (B) LC-MS analysis of Y6 indicating a purity of >95% and an observed m/z of 771.40 for a calculated m/z of 771.41. (C) LC-MS analysis of Y7 indicating a purity of >90% and an observed m/z of 713.40 for a calculated m/z of 713.40. (D) LC-MS analysis of Y8 indicating a purity of >95% and an observed m/z of 713.40 for a calculated m/z of 731.40.

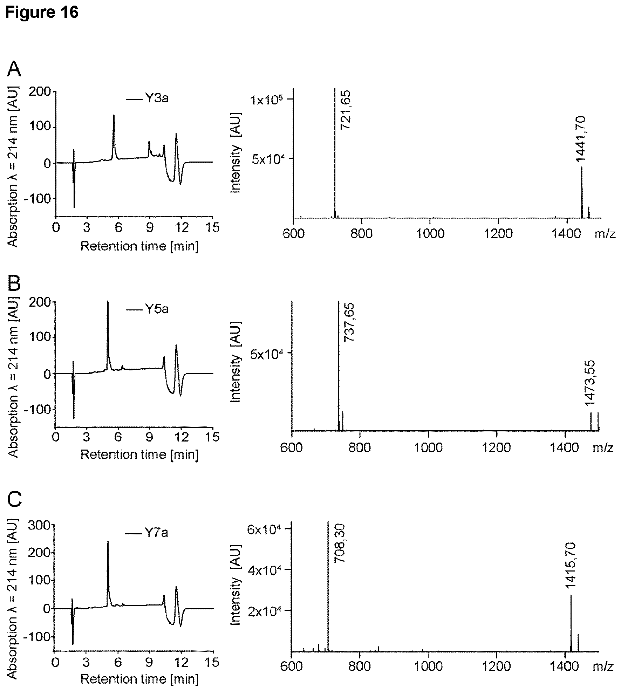

Figure 16 shows a characterization of Y3a, Y5a and Y7a. (A) HPLC chromatogram of Y3a indicating a purity of >60 %. LC-MS analysis of Y3a indicating an observed m/z of 1441.70 for a calculated m/z of 1441.71. (B) HPLC chromatogram of SY5 indicating a purity of >95 %. LC-MS analysis of SY5 indicating an observed m/z of 1473.55 for a calculated m/z of 1473.70. (C) HPLC chromatogram of SY7 indicating a purity of >95 %. LC-MS analysis of SY7 indicating an observed m/z of 1415.70 for a calculated m/z of 1415.69.

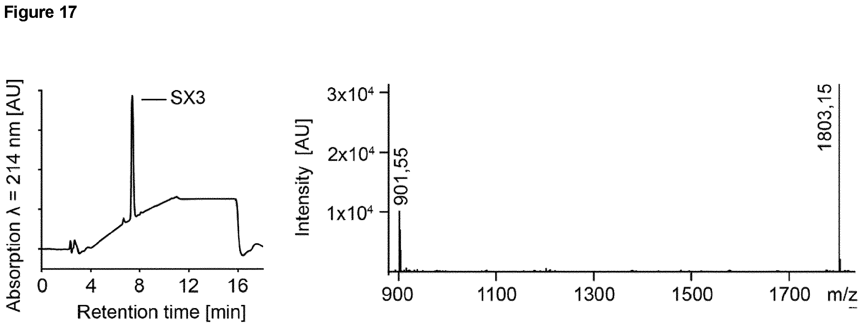

Figure 17 depicts a characterization of SX3. (A) LC-MS analysis of SX3 indicating a purity of >90% and an observed m/z of 1803.15 for a calculated m/z of 1802.85.

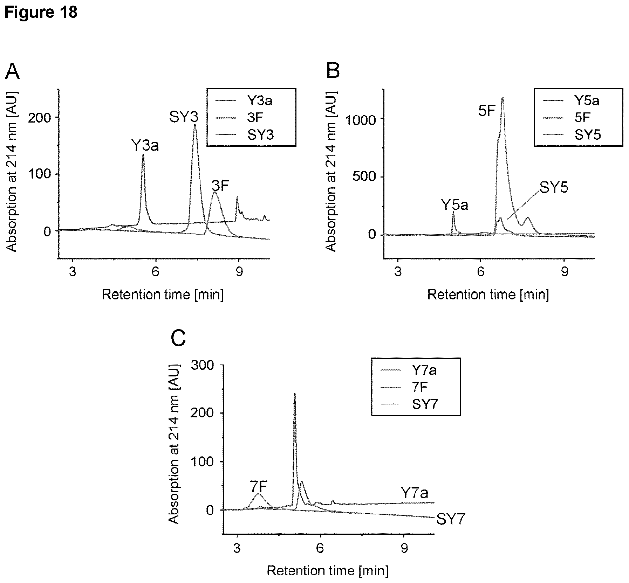

Figure 18 shows an HPLC characterization of SY3, SY5 and SY7. (A) HPLC chromatogram of Y3a, 3F (DBCO-Sulfo-Cy5, red) and SY3. (B) HPLC chromatogram of Y5a, 5F (AFDye488-DBCO) and SY5. (C) HPLC chromatogram of Y7a, 7F (Sulfo-Cy-3-Alkin) and SY7.

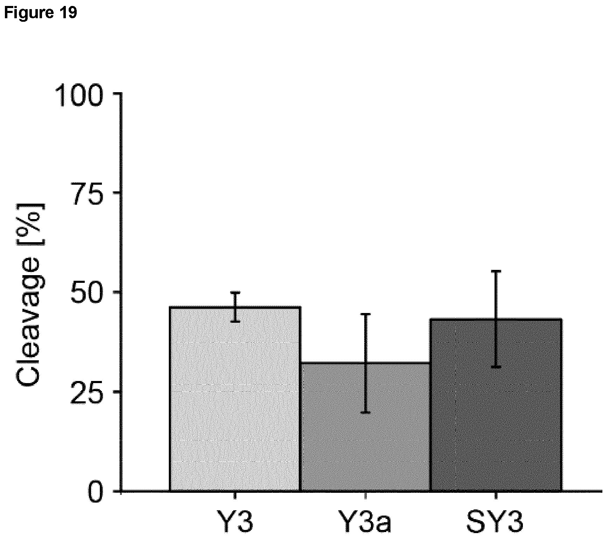

Figure 19 shows cleavage for different sensor stages. (A) Cleavage results for Y3, Y3a, and SY3 with 17.5 nM MMP-8 for 15 min at 37 °C. Experiments were performed with n = 3

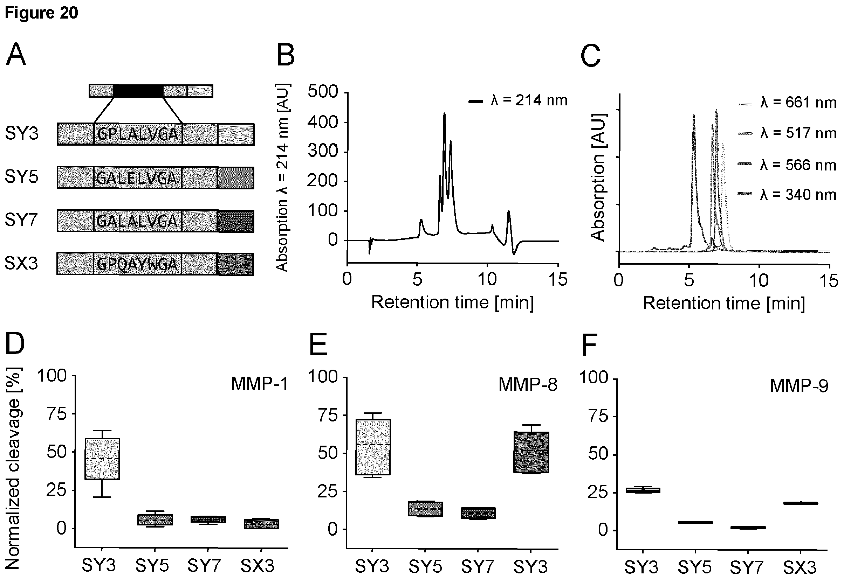

Figure 20 depicts a characterization of multiplex capable fluorescent sensors in mixture. (A) Overview sensors. (B) HPLC chromatogram acquired at λ = 214 nm with SY3, SY5, SY7 and SX3 in mixture. (C) HPLC chromatogram acquired at individual wavelengths for SY3, SY5, SY7 and SX3 in mixture. (D) Normalized MMP-1 cleavage for SY3, SY5, SY7 and SX3 in mixture. (E) Normalized MMP-8 cleavage for SY3, SY5, SY7 and SX3 in mixture. (F) Normalized MMP-9 cleavage for SY3, SY5, SY7 and SX3 in mixture. Box plots show the mean as dotted line, whiskers indicate outliers and the range of the box range indicates 25% and 75% percentiles, respectively.

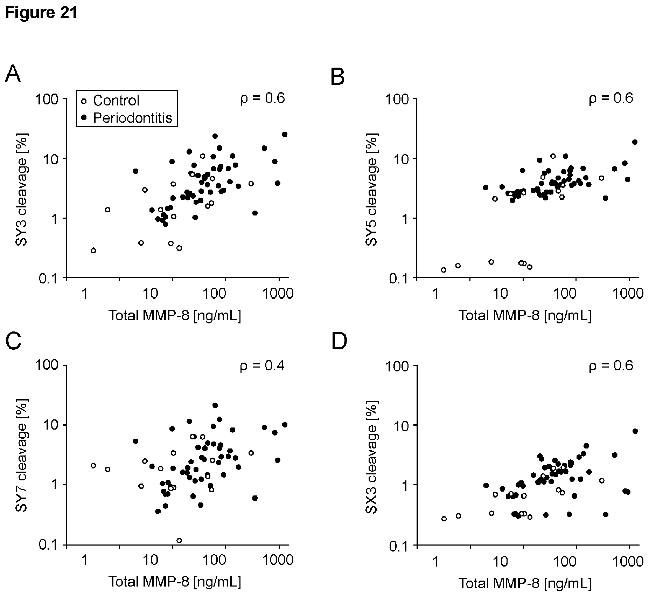

Figure 21 shows the cleavage of individual sensors in human saliva shown with corresponding total MMP-8 concentration determined via ELISA. Data presented in double logarithmic scale, with n indicating the amount of presented data points. (A) SY3 cleavage shown with corresponding total MMP-8 concentration. (B) SY5 cleavage shown with corresponding total MMP-8 concentration. (C) SY7 cleavage shown with corresponding total MMP-8 concentration. (D) SX3 cleavage shown with corresponding total MMP-8 concentration.

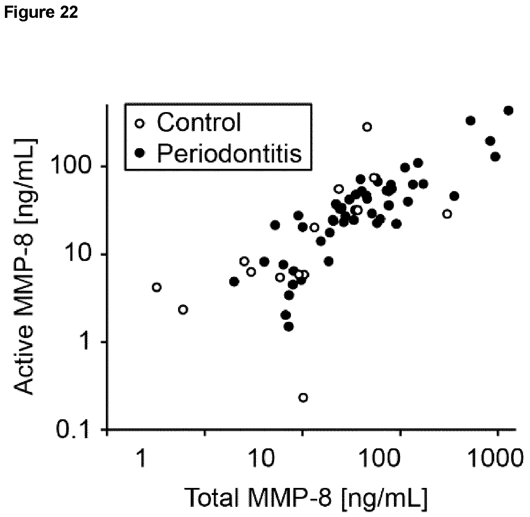

Figure 22 depicts active MMP-8 concentration against total MMP-8 concentration. Active MMP-8 was determined via MMP-8 activity assay and total MMP-8 was determined via ELISA. Data presented in double logarithmic scale.

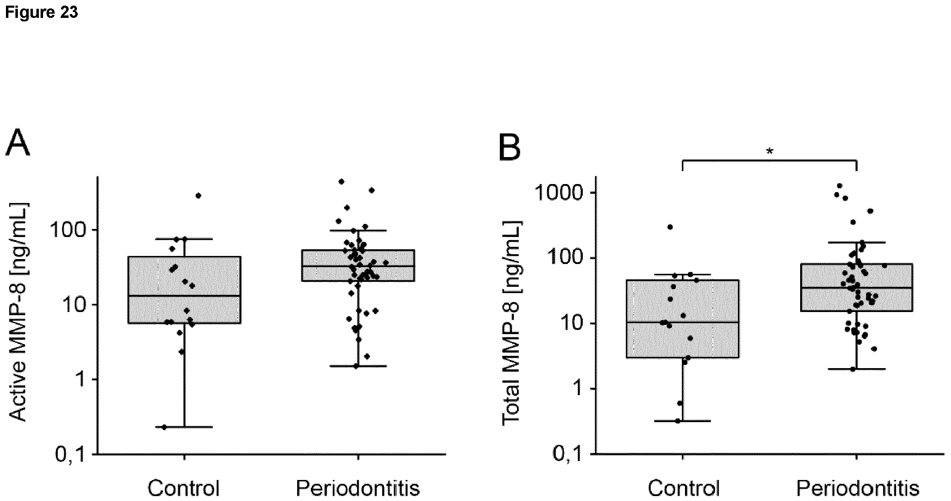

Figure 23 shows MMP-8 concentrations for healthy control and periodontitis group. Data presented in logarithmic scale with box plots showing the median as solid line, whiskers as outliers, and the range of the box range as 25% and 75% percentiles, respectively. (A) Active MMP-8 concentration for healthy control and periodontitis group. (B) Total MMP-8 concentration for healthy control and periodontitis group. P-values <0.05 were considered significant and displayed as * for values <0.05, ** for values <0.005 and *** for values <0.001.

### DETAILED DESCRIPTION

In view of the above objections, the present inventors developed advantageous protease sensitive peptides, which may e.g. be used as peptide sensors that can be formulated e.g., into chewing gums for the lasting presentation of the sensors in the oral cavity. The peptides and peptide sensors, respectively, are cleaved by (upregulated) matrix metalloproteinases (MMP) as present in oral inflammation. Without intending to be bound by theory, this degradation includes the release of flavor substances through which the patient "tastes" inflammation. When tested in patient saliva, sensor cleavage correlated with MMP concentration and activity. Furthermore, the present inventors found that the sensors significantly differentiated patient pools and pools of healthy volunteers, and cleavage was linked to pocket probing depths, a clinical sign of periodontal diseases.

In view of the above, in a first aspect, the present invention relates to a protease-sensitive peptide Pep, wherein said protease-sensitive peptide Pep comprises amino acid residues having the sequence P4-P3-P2-P1-P1'-P2'-P3'-P4', wherein P4 is G, P3 is independently selected from A and P, P2 is independently selected from L and Q, P1 is independently selected from G, A and E, P1' is independently selected from I, L and Y, P2' is independently selected from V and W, P3' is G, and P4' is A, and wherein the said peptide has a length of from 8 to 16 amino acids. Preferably, the length of the peptide is 8 amino acids. Preferably herein, P1 is independently selected from A and E.

In a second aspect, the present invention relates to a compound having the structure Den-R-NH-Pep, or a salt thereof, wherein Den is a detectable label, preferably an optionally substituted denatonium residue, R is an optionally substituted C₁₋₃ alkylene group, Pep is a protease-sensitive peptide, wherein the C-terminus of said peptide forms an amide bond with the group -NH-, and wherein said protease-sensitive peptide Pep comprises amino acid residues having the sequence P4-P3-P2-P1-P1'-P2'-P3'-P4', wherein P4 is G, P3 is independently selected from A and P, P2 is independently selected from L and Q, P1 is independently selected from G, A and E, P1' is independently selected from I, L and Y, P2' is independently selected from V and W, P3' is G, and P4' is A. Preferably herein, P1 is independently selected from A and E.

In the present invention, amino acids are abbreviated as follows: alanine (A), arginine (R), asparagine (N), aspartic acid (D), cysteine (C), glutamine (Q), glutamic acid (E), glycine (G), histidine (H), isoleucine (I), leucine (L), lysine (K), methionine (M), phenylalanine (F), proline (P), serine (S), threonine (T), tryptophan (W), tyrosine (Y) and valine (V).

As will readily be understood by the skilled reader, the amino acid directly attached to the nitrogen atom of the amino-modified denatonium, i.e. the C-terminal amino acid of the protease- sensitive peptide

In one embodiment, the compound or salt of the second aspect has the formula [I]: 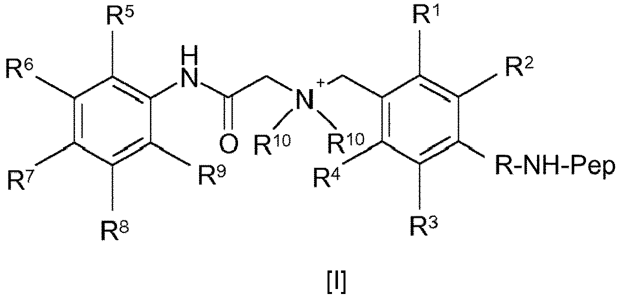 wherein R¹⁰ is hydrogen or C₁-C₁₀-alkyl, and R¹-R⁹ independently are selected from the group consisting of hydrogen, halogen, C₁-C₅-alkyl, C₁-C₄-alkoxy, and C₁-C₂₀-alkoxycarbonyl.

More preferably, R¹⁰, R⁵ and R⁹ is C₁-C₄-alkyl, R¹-R⁴ and R⁶-R⁸ is hydrogen.

In further preferred embodiments, R¹⁰ is ethyl, R⁵ and R⁹ is methyl, and R¹-R⁴ and R⁶-R⁸ is hydrogen.

In one particular embodiment, the compound or salt of the second aspect has the formula [Ia]:

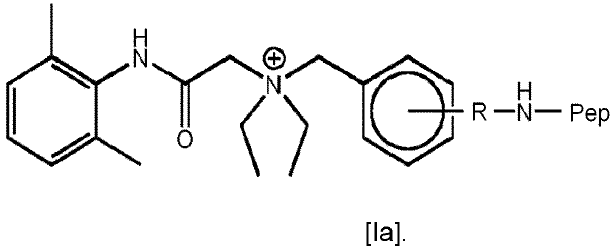

In a more particular embodiment, the compound or salt of the second aspect has the formula [Ib]:

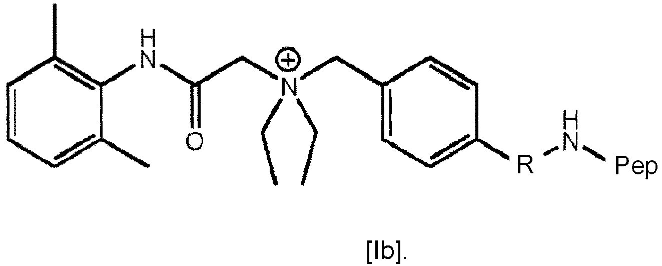

In other embodiments herein, the group -R-NH-Pep may be positions in ortho- or meta position.

In particular embodiments of the first and second aspects, particularly of the second aspect, the protease-sensitive peptide comprises at least 8 amino acids, preferably 8 to 24 amino acids, more preferably 8 to 16 amino acids. Preferably, the protease-sensitive peptide consists of 8 amino acids, preferably of 8 to 24 amino acids, more preferably of 8 to 16 amino acids.

In particular embodiments of the first and second aspects, wherein said protease-sensitive peptide comprises amino acid residues having the sequence P4-P3-P2-P1-P1'-P2'-P3'-P4', wherein P4 is G, P3 is independently selected from A and P, P2 is L, P1 is independently selected from A and E, P1' is independently selected from I and L, P2' is V, P3' is G, and P4' is A.

In further particular embodiments herein, P3 is P.

In further particular embodiments herein, P1 is A.

In other particular embodiments of the first and second aspects, said protease-sensitive peptide comprises amino acid residues having a sequence selected from GPQAYWGA and GPLAYWGA.

In particularly preferred embodiments of the first and second aspects, said protease-sensitive peptide comprises amino acid residues having a sequence selected from the group consisting of GPLELVGA (SEQ ID NO: 2), GPLEIVGA (SEQ ID NO: 3), GPLALVGA (SEQ ID NO: 4), GPLAIVGA (SEQ ID NO: 5), GALELVGA (SEQ ID NO: 6), GALEIVGA (SEQ ID NO: 7), GALALVGA (SEQ ID NO: 8), GALAIVGA (SEQ ID NO: 9), GPQAYWGA (SEQ ID NO: 10) and GPLAYWGA (SEQ ID NO: 11), and even more preferably selected from the group consisting of GPLELVGA (SEQ ID NO: 2), GPLEIVGA (SEQ ID NO: 3), GPLALVGA (SEQ ID NO: 4), GPLAIVGA (SEQ ID NO: 5), and GALALVGA (SEQ ID NO: 8), and GPQAYWGA (SEQ ID NO: 10).

Accordingly, in one embodiment, the said protease-sensitive peptide comprises amino acid residues having a sequence selected from the group consisting of GPLELVGA (SEQ ID NO: 2), GPLEIVGA (SEQ ID NO: 3), GPLALVGA (SEQ ID NO: 4), GPLAIVGA (SEQ ID NO: 5), GALELVGA (SEQ ID NO: 6), GALEIVGA (SEQ ID NO: 7), GALALVGA (SEQ ID NO: 8), GALAIVGA (SEQ ID NO: 9), more preferably selected from the group consisting of GPLELVGA (SEQ ID NO: 2), GPLEIVGA (SEQ ID NO: 3), GPLALVGA (SEQ ID NO: 4), GPLAIVGA (SEQ ID NO: 5), GALELVGA (SEQ ID NO: 6), GALEIVGA (SEQ ID NO: 7), GALALVGA (SEQ ID NO: 8), GALAIVGA (SEQ ID NO: 9).

Accordingly, in another embodiment, the said protease-sensitive peptide Pep comprises amino acid residues having a sequence selected from the group consisting of GPQAYWGA (SEQ ID NO: 10) and GPLAYWGA (SEQ ID NO: 11) - and more preferably has a sequence GPQAYWGA (SEQ ID NO: 10).

In certain preferred embodiments of the second aspect, the said protease-sensitive peptide Pep further comprises one or two linker sequences, more preferably two linker sequences, particularly wherein said two linker sequences are designated as L1 and L2.

Preferably, L1 and L2 are positioned on the N- and C-terminal sides, of any amino acid residues of the peptide explicitly defined herein.

Consequently, in preferred embodiments, the peptides herein have a sequence L1-P4-P3-P2-P1-P1'-P2'-P3'-P4'-L2, wherein particular embodiments of any of the residues correspond to those that are defined elsewhere herein..

In one embodiment, each of said linker sequences comprises at least 4 amino acids, preferably 4 to 15 amino acids. Preferably, each of said linker sequences consists of 4-6, preferably of 4 amino acids. In particular, each linker comprises a sequence comprising two glycine and two serine residues. In preferred non-limiting particular embodiments, each linker comprises, preferably consists of, the sequence GSGS.

Preferably herein, and particularly in context with the second aspect, the protease-sensitive peptide comprises a stabilizing N-terminal modification. More preferably, the protease-sensitive peptide is acetylated at its N-terminus.

As to the protease mentioned in context with the second aspect herein, said protease preferably is a pathogen-specific protease. In one embodiment, the pathogen is a bacterial pathogen, preferably a bacterial pathogen capable of causing inflammation in a subject, preferably a human subject, and in particular in the oral cavity of said subject. In another embodiment, the said pathogen is a viral pathogen.

In context with the present invention, the term "subject" may interchangeably be used with the term "patient".

Preferably herein, the protease is an endopeptidase. More preferably, said protease is a matrix metalloproteinase (MM). Even more preferably, the said protease is a matrix metalloproteinase selected from the group consisting of MM-1, MM-8, and MM-9.

Accordingly, in one particular embodiment, the protease is matrix metalloproteinase 8. In one particular embodiment, the protease is matrix metalloproteinase 1. In one particular embodiment, the protease is matrix metalloproteinase 9.

Furthermore, in particular embodiments, the peptides and compounds of the first and second aspects are cleavable by at least one metalloproteinase selected from the group consisting of MM-1, MM-8, and MM-9, preferably wherein the said at least one proteinase includes MM-8.

In certain embodiments herein, the peptide is not selected from the group consisting of QPW (SEQ ID NO: 16), DAPV (SEQ ID NO: 17), GPQGIAGA (SEQ ID NO: 14), and GPQGIAGQ (SEQ ID NO: 15), and preferably is not selected from the group consisting of GPQGIAGA (SEQ ID NO: 14) and GPQGIAGQ (SEQ ID NO: 15).

In certain embodiments herein, the peptide does not comprise QPW (SEQ ID NO: 16) or DAPV (SEQ ID NO: 17), preferably does not comprise QPW (SEQ ID NO: 16) and DAPV (SEQ ID NO: 17).

In certain embodiments herein, the peptide does not comprise GPQGIAGA (SEQ ID NO: 14) or GPQGIAGQ (SEQ ID NO: 15), preferably does not comprise GPQGIAGA (SEQ ID NO: 14) and GPQGIAGQ (SEQ ID NO: 15).

In particular embodiments herein, the peptides and compounds of the first and second aspects are not susceptible to cleavage by an aminopeptidase.

In particular embodiments herein, the peptides and compounds of the first and second aspects are not susceptible to cleavage by an exopeptidase.

In the present invention, counter ions for the compounds of the invention are not particularly limited. In preferred embodiments, the compounds of the second aspects comprise a counter ion selected from the group consisting of TFA⁻, F⁻, Cl⁻, Br⁻, 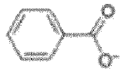 and 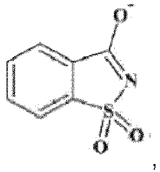 more preferably a counter ion selected from the group consisting of F⁻, Cl⁻, Br⁻, 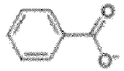 and

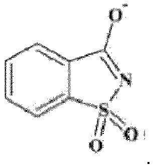

Importantly and generally herein, as the second aspect likewise relates to salts of the compounds of the second aspect, further embodiments and preferred embodiments of the invention correspond to any of the above, yet in the context with the salts of the invention.

Furthermore, in a third aspect of the invention, as will readily be understood by the skilled reader, there is provided a bioresponsive sensor comprising the peptide or compound or salt of the first and second aspects.

A further (fourth) aspect of the present invention provide the peptides, compounds, salts and bioresponsive sensors of the first, second and third aspects for the use in medicine. Consequently, the fourth aspect herein essentially comprises four different variants relating to the peptides, compounds, salts and bioresponsive sensors of the first, second and third aspects, for use in medicine.

Generally, preferred embodiments of the fourth aspect correspond to those of the first, second and third aspects set forth herein.

In an even further (fifth) aspect of the present invention, further provided are peptides, compounds, salts and bioresponsive sensors of the first, second and third aspects for use in a method of detecting a medical condition, particularly an inflammation. Consequently, the fifth aspect herein essentially comprises four different variants relating to the peptides, compounds, salts and bioresponsive sensors of the first, second and third aspects, for use in the said method(s).

In accordance with the invention, the said detecting preferably occurs directly in the oral cavity of the patient by the use of the human tongue (sense of taste), optionally together with the nose (sense of smell) as a detector, and in particular by the sense of taste. If the patient suffers from an inflammation of the oral cavity, the bioresponsive diagnostic sensor comprised e.g. in a chewing gum is considered to come into contact with the saliva containing the matrix metalloproteinases (MMP) long enough to react. Accordingly, it is considered possible for the patient himself to detect an inflammation in the oral cavity, e.g. periodontal disease and/or peri-implantitis by gustatory detection of a bitter taste. Therefore, a person using the inventive chewing gum is considered to know, when detecting a bitter taste, that an inflammation in the oral cavity is present.

Generally, preferred embodiments of the fifth aspect correspond to those of the first, second and third aspects set forth herein.

In particular embodiments, said method is a method of detecting a medical condition, particularly an inflammation in the oral cavity of a human patient.

In particular embodiments herein, inflammation is detected by gustatory perception, preferably by gustatory perception by the patient.

Generally herein, the inflammation is preferably detected upon cleavage of the compounds (salts or bioresponsive sensors) and the subsequent releasing of a denatonium compound.

As already set forth above, in particular embodiments herein, the peptides and compounds of the first and second aspects are not susceptible to cleavage by an aminopeptidase. As opposed to that, after cleavage of the proteolytic peptide by the desired protease, the resulting compound is generally susceptible to cleavage by an aminopeptidase, such that the remaining amino acids of the peptide will subsequently be cleaved off, leaving the detectable denatonium compounds.

In other words, and without intending to be bound by theory, and in line with the findings of the present inventors, inflammation is detectable after cleavage as the resulting compounds are further digested by unspecific aminopeptidase (AP), which ultimately leads to release of denatonium compounds.

Consequently, in one embodiment of the above methods, following cleavage of the compound, a compound having the formula [IIa]: 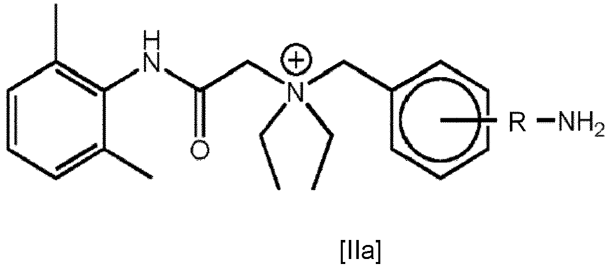 is released, wherein R is an optionally substituted C_{1 3} alkylene group, preferably CH₂.

In a more particular embodiment of the above methods, following cleavage of the compound, a compound having the formula [IIb]: 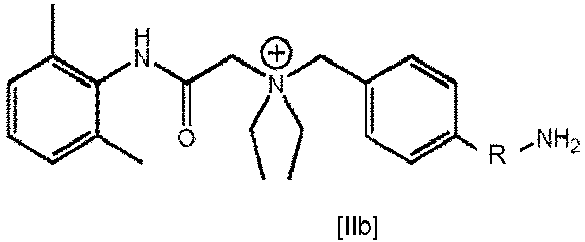 is released, wherein R is an optionally substituted C_{1 3} alkylene group, preferably CH₂.

In a sixth aspect, the present invention provides methods for the preparation of the compounds of the second aspects or of the bioresponsive sensors of the third aspect, respectively, which methods comprise the step of linking an amino-modified denatonium compound to the C-terminus of a protease-sensitive peptide.

Generally, preferred embodiments of the sixth aspect correspond to those of the second and third aspects set forth herein.

Accordingly, as one example in context with the sixth aspect, the amino-modified denatonium compound is a compound of formula [IIa]

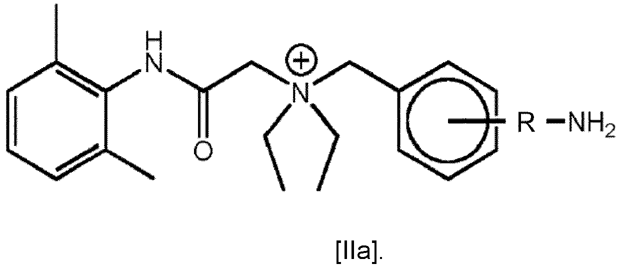

In a more particular embodiment of the sixth aspect, the amino-modified denatonium compound is a compound of formula [IIb]

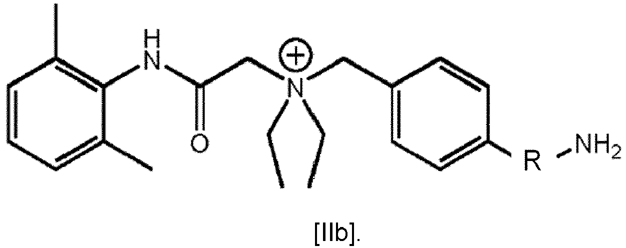

In particular embodiments of the sixth aspect, the methods further comprise, prior to the step of linking, preparing a protease-sensitive peptide by solid phase peptide synthesis.

In preferred embodiments herein, preferably, the methods additionally or alternatively comprise, prior to the step of linking, protecting the N-terminus of the protease-sensitive peptide with a protecting group, preferably acetic anhydride.

In a seventh aspect, there are provided diagnostic chewing gums comprising the peptides, compounds, salts and bioresponsive sensors of the first, second and third aspects.

Generally, preferred embodiments of the seventh aspect correspond to those of the first, second and third aspects set forth herein.

In an eighth aspect, there are provided diagnostic confectionaries comprising the peptides, compounds, salts and bioresponsive sensors of the first, second and third aspects.

Generally, preferred embodiments of the eighth aspect correspond to those of the first, second and third aspects set forth herein.

In a further (ninth) aspects, there are provided the chewing gums of the seventh aspect and the diagnostic confectionary of the eighth aspect for use in medicine.

Generally, preferred embodiments of the ninth aspect correspond to those of the first, second, third and fourth aspects set forth herein.

In a further (tenth) aspects, there are provided the chewing gums of the seventh aspect and the diagnostic confectionary of the eighth aspect for use in a method of detecting a medical condition, particularly an inflammation.

Generally, preferred embodiments of the ninth aspect correspond to those of the first, second, third, fourth and fifth aspects set forth herein.

As will readily be appreciated by the person skilled in the art to which this invention pertains, the present inventors have performed a significant number of experiments as will be set forth in detail from the present Examples.

This will be set forth in further detail by the following observations and conclusions that are based on / drawn from the said experiments, which however are not intended to limit the present invention in any way.

Further details on particular features in context with the present invention may be taken from the following documents, which are incorporated by reference herein: EP application 13710333.9, EP application 17758824.1, EP application 17762078.8, EP application 19708959.2, WO 2019/162289, WO 2020/187750.

### EXAMPLES

The invention will now further be described by reference to the following examples. Importantly, those are intended for further illustrating the invention, but are by no means intended to limit the invention in any way.

### Example 1

Proteomic Identification of Cleavage Sites (PICS):
Identification of protease cleavage sites was performed with minor changes according to PICS protocols described by Schilling et al. [34]. Three different PICS libraries were generated, namely HEK, E. coli 14N and E. coli 15N.

HEK-293 cells were cultured in Dulbecco's modified eagle's medium (10 % fetal bovine serum; 1 % penicillin-streptomycin) and grown to confluence. Upon harvesting, cells were washed with cold phosphate buffered saline (PBS), centrifuged at 1500 g / 4 °C for 10 min. The cell pellet was retained and washed twice with PBS.

E. coli (DH5a) cells were cultured either in lysogeny broth medium (14N E. coli) or Spectra 9 medium (Eurisotop, CGM-3030) (15N E. coli) for 16 h at 37° C. The cell suspension was centrifuged at 4000 g / 4 °C for 20 min. The cell pellet was retained and washed two times with PBS.

All cells were lysed by suspension in lysis buffer (125 mM tris(hydroxymethyl)aminomethane (Tris), 5 % sodium dodecyl sulfate (SDS), 100 mM dithiothreitol (DTT), 1 mM phenylmethylsulfonyl fluoride (PMSF)) followed by three cycles of sonication (Sonopuls HD3100, Bandelin, Berlin, Germany) with 25 % amplitude, 30 s sonication, 90 s pause for 10 min on ice. After sonication, an equal amount of radio-immunoprecipitation-assay-buffer (incl. 6 M urea, 5% SDS) was added, shaken for 90 min at 4° C and finally centrifuged at 20,000 g for 30 min. Protein concentration in the supernatant was determined via bicinchoninic acid assay (BCA).

The proteins were alkylated as described. Briefly, lysates were treated with 20 mM iodoacetamide (IAA) in 100 mM 4-(2-hydroxyethyl)-1-piperazineethanesulfonic acid (HEPES), pH 7.5 for 1h in the dark; IAA was quenched with 5 mM DTT. Samples were then trichloracetic acid (TCA) precipitated (15% TCA, 4°C, 1h) followed by centrifugation at 8,500 g for 1 hour, 4°C. The pellets were washed twice with cold methanol and air-dried. Proteins were solubilized in ice-cold 20 mM NaOH in an ultrasonic bath for 1 h followed by tip sonication as mentioned above. Samples were adjusted to 2 mg/mL concentration in 200 mM HEPES, pH 7.5, centrifuged again for 30 min at 8,500 g and the supernatant was retained.

To generate peptide libraries, lysates were digested with trypsin (pH 8.5, 1:100 protease, 16h 37°C). Digestion was verified via SDS gel, with no major bands >10 kDa. Trypsin was inactivated by heat inactivation at 70 °C for 20 min followed by addition of 1 mM PMSF final concentration. Libraries were adjusted to 1 M guanidine hydrochloride and centrifuged at 4500 rpm, at 4 °C for 1 h; the supernatant was retained.

Peptides were alkylated again: Incubation with 5 mM DTT for 1 h at 37 °, was followed by addition of 40 mM IAA and incubation for 1.5 h at 37° in the dark. IAA was quenched with 15 mM DTT and incubated at 37 °C for 10 min.

Chemical modification was performed as described. Briefly, 30 mM formaldehyde and 30 mM sodium cyanoborohydride were added and allowed to react at 25 °C for 2 h. This step was repeated once for an additional 16 h. After that, 100 mM glycine was added and incubated at 25 °C for 30 min. The sample was acidified with TFA (0.5%) and degassed. All samples were purified by C18 solid phase extraction and lyophilized. The lyophilized samples were resolved in 200 mM HEPES, pH 7.5, final concentrations were adjusted to 2 mg/ml. Aliquots were prepared and stored at -80 °C until further usage

### Example 2

### MMP-8 PICS:

300 µg of peptide libraries were adjusted to 200 mM NaCl, 50 mM HEPES, pH 7.5, 5 mM CaCl2 and 1µM ZnCl2. The peptide libraries were incubated with 5 µg activated MMP-8 (see below), for 16 h at 37 °C.

MMP-8 specific peptide fragments were enriched, following the published protocols. Briefly, the sample was adjusted to pH 7-8, 0.5 mM sulfo-NHS-SS-biotin were added and incubated for 2 h at 25 °C. Biotin-tagged peptide fragments were enriched on high-capacity streptavidin sepharose. Washing and elution was done on spin columns. Removal of unspecifically bound peptides was achieved by washing nine times with wash buffer (50 mM HEPES, pH 7.5, 150 mM NaCl). Target peptides were eluted with two times 500 µL elution buffer (50 mM HEPES, 10 mM DTT, pH 7.5), incubated for 2 h at 25 °C. The column was centrifuged and the flow-through was applied to the resin one more time. Afterwards the samples were purified via solid phase extraction (StrataX, Phenomenex) (Washed with 4-5 mL of 0.4 % formic acid (FA), elution in 1.8 mL 0.4 FA/80 % acetonitrile ACN) and stored at -80 °C until analysis.

### Example 3

### Liquid chromatography - mass spectrometry (LC-MS) / Data Analysis:

Samples were resuspended in loading buffer (0,2 % FA/2 % ACN/98 % H2O) prior to analysis. NanoLC-MS/MS analyses were performed on an LTQ-Orbitrap Velos Pro (Thermo Scientific) equipped with a PicoView Ion Source (New Objective) and coupled to an EASY-nLC 1000 (Thermo Scientific). Peptides were loaded on capillary columns (PicoFrit, 30 cm x 150 µm ID, New Objective) self-packed with ReproSil-Pur 120 C18-AQ, 1.9 µm (Dr. Maisch) and separated with a 120-minute linear gradient from 3% to 30% acetonitrile and 0.1% formic acid and a flow rate of 500 nl/min.

MS scans were acquired in the Orbitrap analyzer with a resolution of 30000 at m/z 400, MS/MS scans were acquired in the Orbitrap analyzer with a resolution of 7500 at m/z 400 using HCD fragmentation with 30% normalized collision energy. A TOP5 data-dependent MS/MS method was used; dynamic exclusion was applied with a repeat count of 1 and an; singly charged precursors were excluded from selection. Minimum signal threshold for precursor selection was set to 50000. Predictive AGC was used with AGC target a value of 1e6 for MS scans and 5e4 for MS/MS scans. Lock mass option was applied for internal calibration in all runs using background ions from protonated decamethylcyclopentasiloxane (m/z 371.10124).

### Example 4

### Data analysis:

Data analysis was performed using PMi-Byos software V3.11 by Protein Metrics inc. (United states). Datafiles were searched, with the following settings: Protease set as trypsin (C-terminal to R, K), semi-specific digest (ragged peptide N-terminus), up to 3 missed cleavages. Precursor Tolerance of 7.5 ppm, fragment mass tolerance of 15 ppm. Carbamidomethyl @ C as fixed modification, dimethyl/methyl/thioacyl @ K as common and thiacyl @ peptide N-term as common (residue from the cleavable enrichment probe). With max allowed common modifications at 3 and rare max set to 1. For 15N samples a fixed modification for each amino acid was used to enable search of heavy labelled peptides (see list below). (The present inventors tested to add modifications for potentially unlabeled, or not fully labelled amino acids (e.g. 15N-remove_Gly / -0,997035 @ G), however due to the needed number of additional modifications - at least 21 and up to 30 to account for partial labeling - the inventors found no improvement in the results generated - Data not shown).

### Data was searched against the following databases:

HEK: Human TREMBL+isoforms (downloaded from uniport)
E. Coli: E. coli Reference proteome (downloaded from uniport)

Search Results were filtered on peptide level to not contain reverse proteins (from decoys) and only contain peptides with a PEP 2D score of <0.1, total score >100 and containing 'N-terminal thioacyl.

The resulting peptide list was submitted to the WEB-PICs application (http://clipserve.clip.ubc.ca/pics) and the results were used to enable design of custom peptides [26].
15N - custom modifications:
15N_Gly / +0.997035 @ G | fixed
15N_Ala / +0.997035 @ A | fixed
15N_Ser / +0.997035 @ S | fixed
15N_Thr / +0.997035 @ T | fixed
15N_Cys / +0.997035 @ C | fixed
15N_Val / +0.997035 @ V | fixed
15N_Leu / +0.997035 @ L | fixed
15N_Ile / +0.997035 @ I | fixed
15N_Met / +0.997035 @ M | fixed
15N_Pro / +0.997035 @ P | fixed
15N_Phe / +0.997035 @ F | fixed
15N_Tyr / +0.997035 @ Y | fixed
15N_Asp / +0.997035 @ D | fixed
15N_Glu / +0.997035 @ E | fixed
15N_Trp / + 1.994070 @ W | fixed
15N_Asn / +1.994070 @ N | fixed
15N_Gln / +1.994070 @ Q | fixed

### Example 5

### Peptide synthesis:

All peptides described in the present examples were synthesized in-house using fluorenylmethoxycarbonyl protecting group (Fmoc) based solid phase peptide synthesis, following established protocols using a polypropylene-reactor (MultiSynTech, Witten, Germany).

Syntheses were performed on 100 µmol loaded 2-Chlorotrityl chloride resin (CTC-Resin). The respective first amino acid was coupled in 5-molar excess in dichlormethane (DCM) / diisopropylethylamine (DIPEA) at 25°C under rigorous shaking for at least 3 hours. Free CTC-binding sites were blocked with methanol for 30 minutes at 25 °C under rigorous shaking. The resin was washed 3 times with DCM and 3 times with dimethylformamide (DMF).

The subsequent amino acids were either coupled via semi-automatic Biotage^{®} Initiator+ SP Wave peptide synthesizer (synthesizer 1) or Liberty Blue^{™} automatic peptide synthesizer (synthesizer 2).

### Synthesizer 1:

500 µmol of the respective Fmoc-protected amino acid DMF containing 0.5 M hydroxybenzotriazole, 0.5 M N,N'-Diisopropylcarbodiimide (DIC) and 0.2 M DIPEA at 50 °C for 10 min followed by three wash steps with DMF. Fmoc deprotection was carried out via incubation in 20 % piperidine in DMF for 3 min at 75 °C followed by four wash steps with DMF.

### Synthesizer 2:

0.2 M of the respective amino acid, in DMF containing 1 M ethyl cyanohydroxyiminoacetate and 0.5 M DIC for 15 s at 75 °C, followed by 110 s at 90 °C. Fmoc deprotection was carried out in DMF with 20 % piperidine at 75 °C for 15 s followed by 50 s at 90 °C.

Completed peptides were washed three times with DCM and diethylether (DEE) each and dried. Cleavage was performed with cleavage solution (trifluoroacetic acid, 2.5 % water, 2.5 % triisopropylsilane, 2.5 % 2,2'-(Ethylendioxy)diethanthiol) for 1 h at room temperature under rigorous shaking. Peptides were precipitated in ice cold DEE, centrifuged and the supernatant collected, repeated three times in total.

N-terminal acetylation of the peptides was done on-resin. 2 mL DMF containing 90 µL of DIPEA and 50 µL of acetic anhydrite were added and allowed to react for 30 min at 25 °C under rigorous shaking. The resin was washed 3 times each with DMF, DCM and DEE and dried.

Preparative peptide purification was carried out using an GE ÄKTA pure (GE Healthcare, Chalfont St Giles, UK) system with a Luna 15 µm C18 100 Å column, 21.2 mm internal diameter, 250 mm length (Phenomenex Inc., Torrance, CA), with eluant A (0.1% trifluoroacetic acid (TFA) in water (v/v)) and eluent B (0.1% TFA in ACN (v/v)) with varying gradients starting from 10-50% (depending on the peptides solubility) to 100 % B in 60 minutes. UV absorption was read at λ = 214 nm 254 nm and 280 nm. The samples were frozen in liquid nitrogen, freeze-dried, and stored at -80 °C until further usage.

### Example 6

### Liquid chromatography-mass spectrometry (LC-MS):

Peptide mass spectra were acquired via a Shimadzu LC-MS system (Shimadzu Scientific Instruments, Columbia, MD, USA) equipped with a DGU-20A3R degassing unit, a LC20AB liquid chromatograph using a Synergi 4 µm fusion-RP column (150 x 4.6 mm) (Phenomenex Inc., Torrance, CA), SPD-20A UV/Vis detector, coupled with a single quadrupole LCMS-2020. Mobile phase A was 0.1 % (v/v) FA in Millipore water. Mobile phase B was methanol with 0.1 % (v/v) FA, flow was set to 1 ml/min, the wavelength of the detector was set to λ = 214 nm and the injected volume of the sample was 20 µL. The gradient was increased in 8 min from 5 to 90 % B, then held at 90 % B for 5 min, reduced to 5 % B within 1 min, and held at 5 % B for 4 min. Detection was done at λ = 214 nm. MS-detection range was set to 60 to 1000 (m/z).

### Example 7

### MMP activation:

Pro-Matrix-metalloproteinases (MMP-1, MMP-8, and MMP-9) (Merck Millipore, Burlington, MA, USA) were activated prior usage. To activate Pro-MMPs a fresh 50 mM 4-Aminophenylmercuric acetate (APMA) stock solution in 0.1 M NaOH was prepared. The stock solution was added in a 10:1 (v/v) ratio to Pro-MMPs (Pro-MMPs:APMA) to reach a final concentration of 5 mM APMA. The solution was incubated for 3 h at 37 °C without agitation. Following incubation, the activated MMPs were stored in aliquots at -80 °C.

### Example 8

### Peptide cleavage:

Lyophilized peptides were solubilized in MMP buffer (200 mM NaCl, 50 mM Tris-HCl, 5 mM CaCl2, 1 µM ZnCl2, 0.05 % Brij35 at pH 6.8-7.0) to 1 mM stock solutions. Cleavage was performed at 37 °C under rigorous shaking in 30 µL MMP buffer containing 0.1 mM of the respective peptide and 900 ng/mL MMP-8. The reaction was stopped by addition of 5 mM EDTA final concentration. Iterative improvement and sensor stage experiments were performed n = 3, and experiments comparing Y1-8 and X3 were performed n = 5.

Analytical HPLC was carried out via an Agilent 1260 infinity II HPLC (Agilent Technologies Inc., Waldbronn, Germany) using a Zorbax 300SB-CN 5µm analytical 4.6 x 150 mm LC column (Agilent Technologies Inc., Waldbronn, Germany) The device was equipped with a diode array detector (G7115A, Agilent), a fluorescence detector (G7121A, Agilent), an automatic vial sampler (G7129C, Agilent), flexible Pump (G7104C, Agilent), and multicolumn oven (G7116A, Agilent). Mobile phase A was 0.1% TFA in Millipore water. Mobile phase B was ACN with 0.1% TFA, flow was set to 1 ml/min, injection volume was 15 µl, and the wavelength of the detector was set to λ = 214 nm for PICS derived peptides.

The gradient was held at 15% B for 1 minute, then increasing to 40% within 7 minutes, increased again for 0.5 minutes to 95% B, held for 0.5 min, then back to 15% B within 0.5 minute, and held for 5.5 minutes.

Cleavage percent was analyzed by comparing the area under the curve (AUC) of the main peak from the compound incubated with MMP and in absence of MMP.

### Example 9

### Denatonium-CH2-NH2 synthesis:

1.5 molar equivalents of lidocaine were mixed with 1 molar equivalent of tert-butyl-4-(bromomethyl)benzylcarbamate without solvents. The mixture was heated to 80 °C under stirring until the formation of a molten mixture and kept at 80 °C for 10 min to obtain a yellow solid. The solid was treated with a mixture of n-hexan and ethyl acetate (1:1) until the precipitation of white solid. The solid was filtrated and washed with a mixture of n-hexan and ethyl acetate (1:1). Afterwards, the solid was dried under vacuum. To remove the tert-butyloxycarbonyl protection-group, the solid was solubilized in pure TFA and shaken at 25 °C for 1 h. The resulting product was precipitated with the addition of DEE with a temperature of -20 °C. The precipitate was air-dried and then purified via reversed phase chromatography.

### Example 10

### Sensor synthesis:

Denatonium labeled sensors were synthesized via coupling of purified, side-chain-protected peptides with 2 molar equivalents of Denatonium-CH2-NH2 and 4 molar equivalents of DIPEA in DMF. The reaction was performed at RT for 16 h under rigorous shaking. The sensors were then precipitated with DEE as described above.

DBCO-functionalized dyes were incubated with 1.5 molar excess of the respective azide-functionalized peptides in 50/50 DMSO/water (v/v) for 16 h under shaking at room temperature. The sensors were then purified by reverse phase purification as described before.

Alkyne-functionalized dyes were incubated with 1.5 molar excess of the respective azide-functionalized peptides in water with 500 µM tris-hydroxypropyltriazolylmethylamine, 100 µM CuSO4 and 5 mM sodium ascorbate. The reaction was incubated for 1 h at room temperature and stopped by adding 500 µM ethylenediaminetetraacetic acid (EDTA). The sensors were then purified via anion exchange chromatography via fast protein liquid chromatography on a GE ÄKTA pure (GE Healthcare, Chalfont St Giles, UK) system with a Hitrap Q FF (GE Healthcare, Chalfont St Giles, UK) column, with eluant A (20 mM Tris HCL pH 8) and eluent B (20 mM Tris HCL pH 8, 1 M NaCl) from 0% B to 100 % B in 60 minutes. Afterwards, the sensor was purified via reverse phase purification as described before.

Sensors were freeze-dried (as described above) and stored at -80 °C until further usage.

### Example 11

### Sensor cleavage:

Lyophilized sensors and Y3 were solubilized in MMP buffer (200 mM NaCl, 50 mM Tris-HCI, 5 mM CaCl2, 1 µM ZnCl2, 0.05 % Brij35 at pH 6.8-7.0) to 1 mM stock solutions. Cleavage was performed at 37 °C under rigorous shaking in 30 µL MMP buffer containing 0.1 mM of the respective sensor/peptide and 900 ng/mL MMP-8, or a molar equivalent of MMP-1 or MMP-9 for single cleavage conditions. Cleavage of sensors in mixture was performed with sensor concentrations of 25 µM for each sensor. The reaction was stopped via addition of 5 mM EDTA final concentration.

Experiments were performed n = 5. Cleavage was analyzed via fluorescent detection via HPLC with following wavelengths: SP3 (absorption: 646 nm; emission: 661 nm), SP5 (absorption: 494 nm; emission: 517 nm), SP7 (absorption: 553 nm; emission: 566 nm) and SN3 (absorption: 280 nm; emission: 350 nm). Y3 cleavage was analyzed as described before. Cleavage percent was analyzed by comparing the AUC of the main peak from the compound incubated with MMP and in absence of MMP. Cleavage percent was then normalized by multiplying cleavage results with a correction factor. The correction factor was determined individually for every experiment/MMP by dividing Y3 cleavage results acquired in protease sensitive linker identification study by cleavage results of Y3 in individual sensor cleavage experiments (Figure 3 D-F).

### Example 12

### Sensor cleavage in human saliva:

Lyophilized sensors were solubilized in MMP buffer to 0.05 mM each and mixed with saliva 1:1 (v/v) to reach a final concentration of 25 µM per sensor. The solution was incubated for 15 min at 37 °C under rigorous shaking and the reaction was stopped via addition of 5 mM EDTA final concentration. The samples were diluted 1:2 with acetonitrile, 1:5 with water (1:10 total dilution) and analyzed via HPLC as described before. Cleavage efficiency was analyzed by comparing the AUC of the newly formed peaks compared to the total AUC of the combined peaks. Peaks with LU < 1 were considered below the limit of detection and set to 0.5 LU for further analysis.

### Aminopeptidase cleavage:

Lyophilized sensors were solubilized in AP buffer (50 mM Tris-HCI, 1mM CaCl2, 150 mM NaCl at pH 7.0) to 1 mM stock solutions. Cleavage was performed at 37 °C under rigorous shaking in 30 µL AP buffer containing 0.1 mM of the respective sensor and 0, 0.87, 8.7, 39 or 78 µg/mL AP for 1 h for concentration-based cleavage. For time resolved cleavage of S1c, cleavage was performed at 37 °C under rigorous shaking in 30 µL AP buffer containing 0.1 mM of the respective sensor and 0.87 µg/mL AP for 0, 5, 10, 20, 30 and 60 min. The reaction was stopped via 15 min incubation at 95 °C and analyzed by HPLC described before. Cleavage was calculated as the decrease of the main peak in relation to a negative control, indicating cleavage as the cleavage of one or more amino acids from the N-terminus. All experiments were performed n = 3.

### Example 13

### Human sample collection and storage:

The study protocol was prepared in accordance with the Declaration of Helsinki and the criteria of good clinical practice. It was approved by the Ethics Committee of the University of Würzburg (file number 190/18). Written informed consent was obtained from all selected patients.

The study participants were recruited from patients visiting the department of Periodontology of the University Hospital of Würzburg, Germany between Oct/2019 to Dec/2020. Periodontally healthy patients as well as patients with periodontal diseases of varying severity (gingivitis, periodontitis) were included. The periodontally diseased patients were in different stages of treatment (untreated patients before active initial therapy, treated recall patients in regular supportive aftercare). Eligibility criteria were age 18 to 99 years. Patients of both sexes, with and without periodontal disease were included in the study.

Periodontal examinations were obtained from all study participants comprising the documentation of missing teeth, probing pocket depths (PPD), recession, bleeding on probing (BoP), clinical attachment level (CAL), furcation involvement, tooth mobility and plaque control record (PlaCR). Demographic details and general health profile of the participants including current intake of medications and frequency of smoking was documented using a questionnaire. CAL, PPD and BoP were recorded at 6 sites per tooth (mesio-buccal, buccal, disto-buccal, disto-oral, oral, mesio-oral) using a pressure calibrated computer aided Florida Probe handpiece. Any bleeding spot appearing within 30 seconds after probing was recorded as a BoP-positive site. Furcation involvement was determined using a manual furcation probe (Nabers Probe). The total amount of periodontal inflammation present in each patient was calculated from the recorded BoP, CAL and PPD scores using the PISA-Index [35]. Collected samples were quality-controlled hosted by the certified centralized biobank of the medical faculty of the University of Würzburg [36].

### Example 14

### Total MMP-8 concentration determination:

Saliva samples were thawed at 4 °C, overnight, diluted 1:200 and analyzed by MMP-8 ELISA (Sigma-Aldrich, Taufkirchen, Germany) following provided protocols. In brief, samples and reagents were brought to room temperature. Samples were distributed on the sample plate, covered, and incubated for 2.5 h at room temperature under mild agitation. The sample plate was washed 4 times with wash buffer, supplied detection antibodies were added, allowed to bind 1 h at room temperature under mild agitation. Four wash steps followed, and finally streptavidin solution was added. The plate was covered and incubated for 45 min at room temperature under mild agitation, washed 4 additional times and detection substrate added, it was covered and incubated for 30 min at room temperature in the dark. The reaction was stopped, and absorbance measured at 450 nm in a microplate reader (Tecan Infinite F50, Crailsheim, Germany). Results were compared against a standard curve as supplied.

### Active MMP-8 concentration determination:

Saliva samples were prepared as above. The MMP-8 activity assay (Quickzyme, Leiden, Netherlands) was performed following provided protocols. In brief, reagents were prepared as described by protocols. A 96 well plate was prepared: anti-MMP-8 antibody solution was added, allowed to bind at 37 °C for 2 h, followed by 4 wash steps, incubation with assay buffer and then addition of either samples (100 µL of diluted saliva) or standards as supplied. The plate was covered and incubated at 4 °C overnight and washed 4 times with wash buffer. 1.5 mM APMA solution was freshly prepared and added to wells containing standards. Samples were treated with assay buffer to measure endogenous MMP-8 levels. The plate was covered and incubated at 37 °C for 1 h, detection reagent freshly prepared and added. Absorbance was measured at 405 nm in a microplate reader (Tecan Infinite F50, Crailsheim, Germany) at 0, 2, 4 and 6 h. Between measurements, the plate was incubated at 37 °C.

### Example 15

### Statistical analysis:

Comparison between two groups and correlations were performed using MEDAS (C. Grund, Margetshöchheim, Germany). For comparisons between two groups, Student's t-test was performed when gaussian distribution was provided and Mann-Whitney U test was used when data was not normally distributed. Correlations were described by analysis of Spearman's rank correlation coefficient (p/rho) when data had no ties and not normally distributed. When data had ties and not normally distributed, Kendall rank correlation (τ/tau) coefficient was used to describe correlations. P-values <0.05 were considered significant and displayed as * for values <0.05, ** for values <0.005 and *** for values <0.001.

### Example 16

To allow the C-terminal coupling of denatonium, an amine-functionalized denatonium derivative (Den-CH2-NH2) was synthesized and characterized (Figure 6) [22]. Sensor S1 and a cut variant (S1c, representing the C-terminal fragment after MMP cleavage) were synthesized and characterized by HPLC and LC-MS, showing purity of >95 % and the predicted molecular mass (Figure 2 A, B, Figure 7 A, B).

Aminopeptidase stability of S1 and S1c was studied for 1 h with aminopeptidase concentrations ranging from 0 to 78 µg/mL, showing no cleavage for all tested concentrations for S1 and a cleavage rate of 95 % at the lowest concentration tested for S1c (Figure 2 C). Cleavage over time was evaluated for S1c with 0.87 µg/mL aminopeptidase over 1 h, indicating a cleavage rate of 95 % after 20 min (Figure 2 D). This confirmed that unspecific aminopeptidase digest of the PSL occurs only after the specific cleavage event.

S1 was then incubated with either 4 nM or 8 nM MMP mix (a mixture of MMPs 1, 8, and 9), resulting in cleavage rates of <1 % and >20 % after 5 min, respectively (Figure 2 E). To test the cleavage of individual MMPs, the sensor was incubated with MMP-1, MMP-8, and MMP-9 for 15 min with concentrations ranging from 1 to 10 nM (Figure 2 F). Cleavage was observed for MMP-1 and MMP-9 but not for MMP-8, with a maximum cleavage of 7 % for MMP-1 and 10 % for MMP-9. Therefore, the present inventors redesigned the PSL to improve the overall MMP sensitivity and MMP-8 susceptibility [13].

### Example 17

For S1 improvement, two different strategies were followed to optimize the PSL of S1 (Figure 1 B, Figure 3 C). The first strategy exchanged individual amino acids of the S1-PSL in an iterative process based on previously published information reporting increased MMP-8 specificity (Figure 3A) [24]. These reports rated tryptophan in position P2' as the most effective means of increasing MMP-8 cleavage rate and suggested placing Tyrosine in P1', Alanine in P1, and Leucine in P2. Consequently, the present inventors replaced Alanine in the S1-PSL with Tryptophan (referred to as PSL-X1) and observed an increase in cleavage. PSL-X1 was further improved by introducing Tyrosine (PSL-X2), and the PSL-X2 was further enhanced by introducing Alanine (PSL-X3).[24] Leucine introduction, resulting in PSL-X4, reduced cleavage compared to PSL-X3. Therefore, PSL-X3 was selected. The second strategy searched alternative PSLs by Proteomic Identification of protease Cleavage Sites (PICS; Figure 3 B, Figures 11-13) [25]. The present inventors generated chemically modified proteome libraries from E. coli, 15N labeled E. coli and human embryonic kidney (HeK) cells. These libraries were incubated with MMP-8, and resulting cleavage products were isolated, enriched, and analyzed by MS. PICS indicated that P3 should be an alanine or proline, P2 a leucine, P1 a glutamic acid or alanine, P1' a leucine or isoleucine, P2' a valine, and P3' an alanine or glycine (Figure 3 B). These amino acids were selected by considering their relative occurrence, as determined through WebPICS [26]. The present inventors set the relative occurrence threshold at 11.5 % or more (average of at least three independent PICS results) to limit the number of possible candidates for peptide design [25]. The present inventors chemically synthesized eight PSLs based on these insights, excluding the P3'glycine variant to avoid structural repetitions. Furthermore, none of the hits exceeded the threshold of 11.5% for P4 or P4', so glycine and alanine were selected for these positions, respectively. In addition, Glycine and alanine are in positions P4 and P4' in the S1-PSL (Table 1). The PICS-derived PSLs are called Y1-PSL to Y8-PSL, were characterized and tested for cleavage rates at 17.5 nM MMP-1, MMP-8, or MMP-9 with an incubation time of 15 minutes (Figures 14, 15; Figure 3 D-F). The present inventors selected three PSLs for further testing. PSL-Y3 was chosen for its high cleavage rate by all MMPs. PSL-Y5 and PSL-Y7 were selected based on cleavage by MMP-8 while not being cleaved by MMP-9. PSL-X3 (see above; optimized from previously reported insights [24]) was chosen due to its high cleavage rate for MMP-8 and low cleavage rate for MMP-1 and MMP-9.

**Table 1. Overview of peptides and sensors detailed herein.**

| SEQ ID | Abbrevation | Sequence | Characterization. |
|---|---|---|---|
| 19 | S0 | Ac-GPQGIAGA-Den | Figure 9 |
| 20 | S1 | Ac-GSGS-GPQGIAGA-GSGS-Den | Figure 7 |
| 21 | S1c | IAGA-GSGS-Den | Figure 7 |
| 22 | S0p | Ac-GPQGIAGA | Figure 9 |
| 23 | S1p | Ac-GSGS-GPQGIAGA-GSGS | Figure 9 |
| 24 | X1 | Ac-GPQGIWGA | Figure 10 |
| 25 | X2 | Ac-GPQGYWGA | Figure 10 |
| 26 | X3 | Ac-GPQAYWGA | Figure 10 |
| 27 | X4 | Ac-GPLAYWGA | Figure 10 |
| 28 | Y1 | Ac-GPLELVGA | Figure 14 |
| 29 | Y2 | Ac-GPLEIVGA | Figure 14 |
| 30 | Y3 | Ac-GPLALVGA | Figure 14 |
| 31 | Y4 | Ac-GPLAIVGA | Figure 14 |
| 32 | Y5 | Ac-GALELVGA | Figure 15 |
| 33 | Y6 | Ac-GALEIVGA | Figure 15 |
| 34 | Y7 | Ac-GALALVGA | Figure 15 |
| 35 | Y8 | Ac-GALAIVGA | Figure 15 |
| 36 | Y3a | Ac-GSGS-GPLALVGA-GSGS-Aha | Figure 16 |
| 37 | Y5a | Ac-GSGS-GALELVGA-GSGS-Aha | Figure 16 |
| 38 | Y7a | Ac-GSGS-GALALVGA-GSGS-Aha | Figure 16 |
| 39 | SY3 | Ac-GSGS-GPLALVGA-GSGS-Aha-DBCO- Sulfo-Cy5 | Figure 18 |
| 40 | SY5 | Ac-GSGS-GALELVGA-GSGS-Aha-AFDye488- DBCO | Figure 18 |
| 41 | SY7 | Ac-GSGS-GALELVGA-GSGS-Aha-Sulfo-Cy-3- Alkin | Figure 18 |
| 42 | SX3 | Ac-GSGS-GPQAYWGA-GSGS-Den | Figure 17 |

| | | | |
|---|---|---|---|
| Sequence Abbreviations: The one letter amino acid code was used, except for N-terminal acetylation (Ac), C-terminal functionalization with Den-CH2-NH2 (Den), L-azidohomoalanine (Aha) and for fluorescent dyes (DBCO-Sulfo-Cy5, AFDye488-DBCO, Sulfo-Cy-3-Alkin) | | | |

These four selected PSLs were then assembled into fully functional sensors using four different fluorophores and referred to as SY3, SY5, SY7, and SX3 in reference to PSL-Y3, PSL-Y5, PSL-Y7, and PSL-X3 (vide supra). SY3, SY5, and SY7 were synthesized with C-terminal L-azidohomoalanine (Aha) and "clicked" to different fluorescent dyes at their respective C-termini. SX3 was modified with the (fluorescent) denatonium derivative. The fluorescence spectra did not overlap. Therefore, the inventors could simultaneously multiplex all sensors under identical conditions (Figure 4 A-C, Figure 16-18). Multiplexing was tested against MMP-1, MMP-8, or MMP-9 (Figure 20 D-F, 17.5 nM; incubation 15 minutes). The cleavage outcome of the sensors generally confirmed the effect observed for the PSL alone (Figure 3 D-F). SY3 was cleaved most effectively by MMP-1 and MMP-8, and, to a lesser extent, MMP-9. SY5 responded to MMP-8 but not MMP-1 or MMP-9, with generally low cleavage rates. SY7 had a low general cleavage rate and responded to all MMPs other than MMP-9. SX3 showed high cleavage rates for MMP-8, low cleavage rates for MMP-1, and medium cleavage rates for MMP-9. Further modifications of the sensors distant from the PSL did not impact cleavage performances (e.g., PSL, linker-PSL-linker, or linker-PSL-linker-payload, Figure 8, 19).

### Example 18

These sensors were subsequently profiled in saliva samples from 16 healthy subjects and 50 established periodontitis patients. The saliva was characterized for total MMP-8 and active MMP-8 and correlated with cleavage rates of the sensors (Figure 5, Figure 21, Tables 2-4). Further patient information was collected, including age, smoking status, BOP, the number of missing teeth, recession, plaque, tooth mobility, furcation involvement, gingival pocket depth, clinical attachment level, periodontal epithelial surface area (PESA), and periodontal inflamed surface area (PISA) separately. The selected cohort consisted of treated and stable patients, and the chewing gum could be profiled in this group for follow-up checks at home.T present inventors chose these patients as they wanted to establish a saliva library with different MMP-8 profiles and test the present sensors on that library accordingly. Sensor cleavage significantly correlated with total and active MMP-8 concentration (Figure 5; Table 4). However, another interesting patient pool is naive periodontitis patients to facilitate diagnosis in early stages. Future studies should address this patient pool. Furthermore, all sensors except SY7 were significantly better cleaved in saliva from patients compared to saliva from healthy donors (Table 5). Lastly, gingival pocket depths significantly correlated with sensor cleavage, except SY7 (Table 6).

**Table 2. Data overview from collected control saliva samples.**

| Patient Status | Patient number | ELISA [ng/mL] | Activity [ng/mL] | Cleavage SY3 f%] | Cleavage SY5 [%] | Cleavage SY7 [%] | Cleavage SX3 [%] |
|---|---|---|---|---|---|---|---|
| Control | **1** | 23.57 | 55.46 | 5.40 | 4.88 | 6.37 | 1.40 |
| Control | **2** | 0.32 | 4.20 | 0.29 | 0.14 | 2.10 | 0.27 |
| Control | **3** | 36.73 | 31.90 | 11.05 | 11.04 | 6.33 | 1.89 |
| Control | **4** | 45.81 | 284.25 | 1.60 | 2.84 | 1.38 | 0.82 |
| Control | **5** | 10.38 | 5.85 | 1.07 | 0.17 | 0.89 | 0.33 |
| Control | **6** | 2.53 | 8.30 | 0.38 | 0.18 | 0.95 | 0.34 |
| Control | **7** | 0.60 | 2.34 | 1.39 | 0.16 | 1.81 | 0.30 |
| Control | **8** | 9.18 | 5.87 | 0.37 | 0.18 | 0.87 | 0.33 |
| Control | **9** | 13.21 | 20.29 | 0.31 | 0.15 | 0.12 | 0.29 |
| Control | **10** | 5.91 | 5.43 | 1.39 | 2.55 | 1.86 | 0.70 |
| Control | **11** | 300.59 | 29.02 | 3.77 | 4.69 | 3.42 | 1.17 |
| Control | **12** | 10.23 | 0.23 | 3.71 | 2.64 | 3.42 | 0.66 |
| Control | **13** | 2.99 | 6.28 | 2.96 | 2.11 | 2.50 | 0.69 |
| Control | **14** | - | 17.80 | 0.34 | 0.17 | 0.13 | 0.31 |
| Control | **15** | 56.04 | 73.70 | 4.60 | 3.56 | 2.58 | 1.95 |
| Control | **16** | 53.85 | 74.59 | 1.80 | 2.25 | 0.83 | 0.73 |

**Table 3. Data chart from collected periodontitis saliva samples.**

| Patient Status | Patient number | ELISA [ng/mL] | Activity [ng/mL] | Cleavage SY3 [%] | Cleavage SY5 [%] | Cleavage SY7 [%] | Cleav-age SX3 |
|---|---|---|---|---|---|---|---|
| Periodontitis | **17** | 91.25 | 22.28 | 2.91 | 3.77 | 1.46 | 0.65 |
| Periodontitis | **18** | 30.17 | 42.06 | 5.20 | 6.20 | 4.24 | 2.04 |
| Periodontitis | **19** | 152.60 | 110.03 | 7.75 | 4.73 | 2.83 | 4.49 |
| Periodontitis | **20** | 111.46 | 97.00 | 6.76 | 4.23 | 3.01 | 2.93 |
| Periodontitis | **21** | 45.13 | 46.71 | 3.62 | 3.28 | 1.46 | 2.23 |
| Periodontitis | **22** | 25.15 | 33.92 | 7.68 | 5.69 | 6.38 | 1.94 |
| Periodontitis | **23** | 6.39 | 7.60 | 0.92 | 1.98 | 1.05 | 0.64 |
| Periodontitis | **24** | 20.75 | 24.16 | 2.58 | 2.43 | 1.32 | 1.20 |
| Periodontitis | **25** | 355.98 | 46.03 | 1.22 | 2.15 | 0.60 | 0.32 |
| Periodontitis | **26** | 59.03 | 67.21 | 6.74 | 4.20 | 4.80 | 2.11 |
| Periodontitis | **27** | 33.41 | 24.73 | 1.98 | 2.72 | 0.46 | 1.13 |
| Periodontitis | **28** | 34.70 | 31.87 | 2.81 | 3.80 | 1.24 | 1.52 |
| Periodontitis | **29** | 27.47 | 27.37 | 1.86 | 2.70 | 1.79 | 1.33 |
| Periodontitis | **30** | 72.92 | 53.17 | 3.46 | 4.51 | 2.36 | 0.32 |
| Periodontitis | **31** | 35.31 | 48.09 | 3.99 | 4.04 | 2.88 | 1.83 |
| Periodontitis | **32** | 39.16 | 71.76 | 4.92 | 3.91 | 2.67 | 2.58 |
| Periodontitis | **33** | 58.13 | 22.70 | 10.78 | 6.04 | 9.54 | 1.99 |
| Periodontitis | **34** | 831.11 | 195.81 | 8.98 | 8.39 | 7.48 | 0.78 |
| Periodontitis | **35** | 7.98 | 4.50 | 1.46 | 2.78 | 0.70 | 0.30 |
| Periodontitis | **36** | 40.47 | 52.47 | 4.66 | 3.78 | 4.09 | 1.48 |
| Periodontitis | **37** | 15.41 | 14.11 | 2.25 | 2.92 | 1.62 | 1.44 |
| Periodontitis | **38** | 19.07 | 17.72 | 2.22 | 2.84 | 1.93 | 1.09 |
| Periodontitis | **39** | 8.15 | 6.44 | 1.46 | 2.33 | 1.08 | 0.99 |
| Periodontitis | **40** | 18.65 | 8.30 | 2.71 | 3.09 | 1.59 | 1.10 |
| Periodontitis | **41** | 134.82 | 62.17 | 11.08 | 6.84 | 8.29 | 3.35 |
| Periodontitis | **42** | 119.64 | 39.76 | 4.06 | 3.83 | 3.71 | 1.24 |
| Periodontitis | **43** | 4.07 | 8.23 | 1.37 | 3.33 | 2.03 | 0.85 |
| Periodontitis | **44** | 527.08 | 335.12 | 14.82 | 6.70 | 9.13 | 3.17 |
| Periodontitis | **45** | 9.72 | 5.07 | 8.89 | 6.30 | 8.65 | 0.96 |
| Periodontitis | **46** | 76.21 | 52.19 | 14.96 | 6.31 | 12.48 | 2.12 |
| Periodontitis | **47** | 80.46 | 62.15 | 7.27 | 6.92 | 4.60 | 2.34 |
| Periodontitis | **48** | 45.83 | 42.79 | 5.46 | 4.73 | 5.00 | 1.69 |
| Periodontitis | **49** | 7.30 | 3.41 | 1.05 | 2.62 | 0.45 | 0.67 |
| Periodontitis | **50** | 173.23 | 63.31 | 3.42 | 3.65 | 1.98 | 1.64 |
| Periodontitis | **51** | 51.53 | 29.36 | 2.71 | 3.62 | 0.96 | 1.64 |
| Periodontitis | **52** | 76.50 | 36.09 | 6.67 | 5.28 | 4.11 | 2.41 |
| Periodontitis | **53** | 2.00 | 4.86 | 6.17 | 3.23 | 5.35 | 0.97 |
| Periodontitis | **54** | 82.19 | 55.41 | 2.78 | 3.55 | 2.96 | 1.24 |
| Periodontitis | **55** | 22.06 | 37.13 | 5.65 | 3.80 | 2.44 | 2.71 |
| Periodontitis | **56** | 932.89 | 129.81 | 3.84 | 4.46 | 2.59 | 0.76 |
| Periodontitis | **57** | 62.83 | 25.30 | 23.56 | 10.98 | 21.52 | 1.67 |
| Periodontitis | **58** | 7.22 | 1.51 | 0.79 | 2.35 | 0.70 | 0.33 |
| Periodontitis | **59** | 20.46 | 24.85 | 13.10 | 9.35 | 11.59 | 3.03 |
| Periodontitis | **60** | 26.40 | 23.26 | 1.03 | 2.21 | 1.17 | 0.32 |
| Periodontitis | **61** | 1282.65 | 437.77 | 25.35 | 18.84 | 10.19 | 7.92 |
| Periodontitis | **62** | 6.74 | 2.03 | 1.13 | 2.57 | 0.78 | 0.33 |
| Periodontitis | **63** | 5.24 | 21.62 | 0.97 | 2.60 | 0.37 | 0.64 |
| Periodontitis | **64** | 10.07 | 20.56 | 2.17 | 2.84 | 1.89 | 0.65 |
| Periodontitis | **65** | 24.22 | 32.93 | 2.34 | 2.44 | 0.65 | 1.12 |
| Periodontitis | **66** | 9.08 | 27.73 | 1.51 | 2.75 | 0.93 | 1.08 |

**Table 4. Correlations of active and total MMP-8 concentration with individual sensor cleavage.**

| Input 1 | Input 2 | n | ρ/rho | p(ρ/rho) | |
|---|---|---|---|---|---|
| Active MMP-8 | SY3 | 65 | 0.5874 | 0.00000*** | |
| Active MMP-8 | SY5 | 65 | 0.6007 | 0.00000*** | |
| Active MMP-8 | SY7 | 65 | 0.4248 | 0.00038*** | |
| Active MMP-8 | SX3 | 65 | 0.6403 | 0.00000*** | |
| Total MMP-8 | SY3 | 66 | 0.6190 | 0.00000*** | |
| Total MMP-8 | SY5 | 66 | 0.6475 | 0.00000*** | |
| Total MMP-8 | SY7 | 66 | 0.4447 | 0.00021*** | |
| Total MMP-8 | SX3 | 66 | 0.5612 | 0.00000*** | |

| | | | | | |
|---|---|---|---|---|---|
| Spearman's rank correlation coefficient displayed as rho and p-value as p (rho). P-values <0.05 were considered significant and displayed as * for values <0.05, ** for values <0.005 and *** for values <0.001. | | | | | |

**Table 5. Observed differences for different groups.**

| Parameter | Groups | n groups, | p(t) | p(u) |
|---|---|---|---|---|
| Total MMP-8 | Periodontitis vs. control | (50/15) | 0.23 | 0.024 * |
| Active MMP-8 | Periodontitis vs. control | (50/16) | 0.52 | 0.087 |
| SY3 | Periodontitis vs. control | (50/16) | 0.042 * | 0.0072 ** |
| SY5 | Periodontitis vs. control | (50/16) | 0.011 * | 0.00061 *** |
| SY7 | Periodontitis vs. control | (50/16) | 0.13 | 0.17 |
| SX3 | Periodontitis vs. control | (50/16) | 0.012 * | 0.0015 ** |
| Total MMP-8 | Sex (m/f) | (18/31) | 0.012* | 0.14 * |
| Active MMP-8 | Sex (m/f) | (18/31) | 0.023 * | 0.17 * |

| | | | | |
|---|---|---|---|---|
| Student's t-tests p-value displayed as p(t) and Mann-Whitney U tests p value displayed as p(U). P-values <0.05 were considered significant and displayed as * for values <0.05, ** for values <0.005 and *** for values <0.001. | | | | |

**Table 6. Observed correlations within periodontitis group for clinical parameters, MMP-8 concentration, and individual sensor cleavage.**

| Input 1 | Input 2 | n | τ/tau | p(τ/tau) | ρ/rho | p(ρ/rho) |
|---|---|---|---|---|---|---|
| Total MMP-8 | Aqe | 49 | 0.2867 | 0.0037 ** | | |
| Active MMP-8 | Age | 49 | 0.2401 | 0.015 * | | |
| SY3 | Pocket depth | 49 | 0.2237 | 0.023 * | | |
| SY5 | Pocket depth | 49 | 0.2254 | 0.022 * | | |
| SY7 | Plaque | 49 | | | -0.2307 | 0.019 * |
| SY7 | Pocket depth | 49 | 0.1914 | 0.052 | | |
| SX3 | Pocket depth | 49 | 0.2152 | 0.029 * | | |

| | | | | | | |
|---|---|---|---|---|---|---|
| Kendall rank correlation coefficient displayed as tau and p-value as p(tau), Spearman's rank correlation coefficient displayed as rho and p-value as p (rho). P-values <0.05 were considered significant and displayed as * for values <0.05, ** for values <0.005 and *** for values <0.001. | | | | | | |

### Discussion

Generally, the present inventors succeeded in optimizing previously reported sensors for salivary stability (acetylated N-terminus) and cleavage in clinical saliva samples. Furthermore, a storage-stable C-terminal denatonium derivative was introduced (Figure 1 A) [13, 22, 23]. Among others, these modifications substantially simplified previous designs using beads and removed previous challenges on salivary stability and storage stability [13, 23]. The present inventors did optimize sensors in vitro using MMP-8, a key surrogate marker in periodontitis, but selected the final sensors from responses observed in saliva samples collected from diagnosed patients [13].

In addition, as shown by the present inventors, the chewing gum sensors comprising peptides of the invention were stable against unspecific salivary aminopeptidases.

Moreover, following MMP-cleavage of their protease-sensitive linkers (PSL), they rapidly disintegrated, including the release of a bitter flavor (Figure 2 C, D). The new soluble design replaces previous particle-based sensors [13, 23]. One challenge that was solved during replacement was that a particular PSL used in certain previous particle-based design was unresponsive to MMP-8 when integrated into the new and soluble sensors. Therefore, the present inventors initiated a search for novel MMP-8 responsive PSLs compatible with the new and soluble setup (Figure 3). Identified hits were assessed, and candidates individually encoded with fluorescence dyes, multiplexed in patient saliva and control saliva. Sensor cleavage correlated with MMP-8 (total and active) levels in vitro and MMP-8 levels measured in patient saliva (Figure 5).

Furthermore, it was confirmed that cleavage rates were significantly higher in patient saliva compared to saliva from healthy donors (Table 5).

Further adaptations were achieved. For example, the particle in the old design protected the N-terminus of the senor from unspecific cleavage by salivary aminopeptidases. The same was achieved in the new soluble sensor by N-terminal acetylation.

As a result, replacing the old, particle-based design reduces the costs of goods for this high volume, commodity product, facilitates manufacturing, including the integration into chewing gums, and reduces the effort for quality control reflecting the soluble state of the new sensors.

Contrasting previous studies linking salivary MMP-8 to periodontitis, the patient pool used for Figure 23, Table 5 did not show these correlations [27]. Possibly, these differences reflect the used inclusion/exclusion criteria, recruiting treated periodontitis patients in regular supportive aftercare with comparatively low BoP-values ("bleeding on probing"). Treatment reduces acute inflammatory processes and MMP levels [28, 29]. Furthermore, the selected criteria did not gate salivary collection regiments, such as the time of the day or last food/drink intake. MMP-8 is not the only marker correlating with periodontitis [18-21]. Therefore, the possible promiscuity of the present sensors might be advantageous, which is why the present inventors ultimately screened in patient saliva to reflect the complexity in periodontitis. Some sensors which were not exceptionally responsive to MMP-8 (SY3, SY5, and SX3) discriminated against periodontitis, supporting final screenings in biological fluids. The patient pool donated saliva with a relevant range of active inflammation, but this patient pool is not preferably the one targeted by the product. A preferred (future) target profile is the broad, un-diagnosed population to early segment subjects at risk of developing periodontitis.

In any case, the clinical correlation demonstrated further advantageous potential. For example, sensor cleavage correlated with pocket probing depth, an important factor for disease progression [30]. Particularly if corroborated by future clinical studies using pocket depth as the primary endpoint, this evidence further strengthens the approach for community screening and targeted dental diagnosis and dental care in prescreened and enriched patient pools.

**Table 7. Overview of the sequences shown in the sequence listing**

| SEQ ID NO: | Name | Sequence | Comment |
|---|---|---|---|
| 1 | Seq1 | G P3   P2 P1   P1′   P2′ G   A G P/A L   E/A I/L   V   G   A 1 2   3   4   5   6   7   8 | Consensus sequence of claim 5 |
| | | | GXLXJVGA |
| 2 | Seq2 | GPLELVGA | claim 6, cf. Y1 |
| 3 | Seq3 | GPLEIVGA | claim 6, cf. Y2 |
| 4 | Seq4 | GPLALVGA | claim 6, cf. Y3 |
| 5 | Seq5 | GPLAIVGA | claim 6, cf. Y4 |
| 6 | Seq6 | GALELVGA | claim 6, cf. Y5 |
| 7 | Seq7 | GALEIVGA | claim 6, cf. Y6 |
| 8 | Seq8 | GALALVGA | claim 6, cf. Y7 |
| 9 | Seq9 | GALAIVGA | claim 6, cf. Y8 |
| 10 | Seq10 | GPQAYWGA | claims 5, 6; cf. X3 |
| 11 | Seq11 | GPLAYWGA | claims 5, 6; cf. X4 |
| 12 | Seq12 | GPQGIWGA | Figure 10; cf. X1 |
| 13 | Seq13 | GPQGYWGA | Figure 10; cf. X2 |
| 14 | Seq14 | GPQGIAGA | |
| 15 | Seq15 | GPQGIAGQ | |
| 16 | Seq16 | QPVV | |
| 17 | Seq17 | DAPV | |
| 18 | Seq18 | GSGS | Linker |
| 19 | S0 | Ac-GPQGIAGA-Den | Figure 9, Table 1 |
| 20 | S1 | Ac-GSGS-GPQGIAGA-GSGS-Den | Figure 7, Table 1 |
| 21 | S1c | IAGA-GSGS-Den | Figure 7, Table 1 |
| 22 | S0p | Ac-GPQGIAGA | Figure 9, Table 1 |
| 23 | S1p | Ac-GSGS-GPQGIAGA-GSGS | Figure 9, Table 1 |
| 24 | X1 | Ac-GPQGIWGA | Figure 10, Table 1 |
| 25 | X2 | Ac-GPQGYWGA | Figure 10, Table 1 |
| 26 | X3 | Ac-GPQAYWGA | Figure 10, Table 1 |
| 27 | X4 | Ac-GPLAYWGA | Figure 10, Table 1 |
| 28 | Y1 | Ac-GPLELVGA | Figure 14, Table 1 |
| 29 | Y2 | Ac-GPLEIVGA | Figure 14, Table 1 |
| 30 | Y3 | Ac-GPLALVGA | Figure 14, Table 1 |
| 31 | Y4 | Ac-GPLAIVGA | Figure 14, Table 1 |
| 32 | Y5 | Ac-GALELVGA | Figure 15, Table 1 |
| 33 | Y6 | Ac-GALEIVGA | Figure 15, Table 1 |
| 34 | Y7 | Ac-GALALVGA | Figure 15, Table 1 |
| 35 | Y8 | Ac-GALAIVGA | Figure 15, Table 1 |
| 36 | Y3a | Ac-GSGS-GPLALVGA-GSGS-Aha | Figure 16, Table 1 |
| 37 | Y5a | Ac-GSGS-GALELVGA-GSGS-Aha | Figure 16, Table 1 |
| 38 | Y7a | Ac-GSGS-GALALVGA-GSGS-Aha | Figure 16, Table 1 |
| 39 | SY3 | Ac-GSGS-GPLALVGA-GSGS-Aha- DBCO-Sulfo-Cy5 | Figure 18, Table 1 |
| 40 | SY5 | Ac-GSGS-GALELVGA-GSGS-Aha- AFDye488-DBCO | Figure 18, Table 1 |
| 41 | SY7 | Ac-GSGS-GALELVGA-GSGS-Aha- Sulfo-Cy-3-Alkin | Figure 18, Table 1 |
| 42 | SX3 | Ac-GSGS-GPQAYWGA-GSGS-Den | Figure 17, Table 1 |

### LIST OF REFERENCES

### Patent Documents

EP patent application 13710333.9
EP patent application 17758824.1
EP patent application 17762078.8
EP patent application 19708959.2
US 9,526,803 B1
WO 2013/131993
WO 2019/162289
WO 2020/187750

### Non-Patent Documents

[1] R. Brignardello-Petersen, Tooth loss, periodontal disease, and dental caries may be associated with decreased oral health-related quality of life, but there is no evidence about the magnitude of this association, The Journal of the American Dental Association, 148 (2017) e150.
[2] E.D. Roumanas, The social solution-denture esthetics, phonetics, and function, J Prosthodont, 18 (2009) 112-115.
[3] M.A. Nazir, Prevalence of periodontal disease, its association with systemic diseases and prevention, Int J Health Sci (Qassim), 11 (2017) 72-80.
[4] P.I. Eke, W.S. Borgnakke, R.J. Genco, Recent epidemiologic trends in periodontitis in the USA, Periodontology 2000, 82 (2020) 257-267.
[5] W. He, D. Goodkind, P.R. Kowal, An aging world: 2015, in, United States Census Bureau Washington, DC, 2016.
[6] P. Madianos, W. Papaioannou, D. Herrera, M. Sanz, A. Baeumer, A. Bogren, P. Bouchard, M. Chomyszyn-Gajewska, K. Demirel, R. Gaspersic, M. Giurgiu, F. Graziani, K. Jepsen, S. Jepsen, T. O'Brien, I. Polyzois, P.M. Preshaw, M. Rakic, M. Reners, N. Rincic, A. Stavropoulos, S. Sütcü, C. Verner, J.C. Llodra, EFP Delphi study on the trends in Periodontology and Periodontics in Europe for the year 2025, Journal of Clinical Periodontology, 43 (2016) 472-481.
[7] P.M. Bartold, T.E. Van Dyke, An appraisal of the role of specific bacteria in the initial pathogenesis of periodontitis, Journal of Clinical Periodontology, 46 (2019) 6-11.
[8] A. Savage, K.A. Eaton, D.R. Moles, I. Needleman, A systematic review of definitions of periodontitis and methods that have been used to identify this disease, Journal of Clinical Periodontology, 36 (2009) 458-467.
[9] D.F. Kinane, P.G. Stathopoulou, P.N. Papapanou, Periodontal diseases, Nature Reviews Disease Primers, 3 (2017) 17038.
[10] A. Saito, M. Kikuchi, F. Ueshima, S. Matsumoto, H. Hayakawa, H. Masuda, T. Makiishi, Assessment of oral self-care in patients with periodontitis: a pilot study in a dental school clinic in Japan, BMC Oral Health, 9 (2009) 27.
[11] S. Kc, X.Z. Wang, J.E. Gallagher, Diagnostic sensitivity and specificity of host-derived salivary biomarkers in periodontal disease amongst adults: Systematic review, Journal of Clinical Periodontology, 47 (2020) 289-308.
[12] N. Arias-Bujanda, A. Regueira-Iglesias, C. Balsa-Castro, L. Nibali, N. Donos, I. Tomás, Accuracy of single molecular biomarkers in saliva for the diagnosis of periodontitis: A systematic review and meta-analysis, Journal of Clinical Periodontology, 47 (2020) 2-18.
[13] J. Ritzer, T. Lühmann, C. Rode, M. Pein-Hackelbusch, I. Immohr, U. Schedler, T. Thiele, S. Stübinger, B.v. Rechenberg, J. Waser-Althaus, F. Schlottig, M. Merli, H. Dawe, M. Karpisek, R. Wyrwa, M. Schnabelrauch, L. Meinel, Diagnosing peri-implant disease using the tongue as a 24/7 detector, Nature Communications, 8 (2017) 264.
[14] A. Brockhoff, M. Behrens, A. Massarotti, G. Appendino, W. Meyerhof, Broad tuning of the human bitter taste receptor hTAS2R46 to various sesquiterpene lactones, clerodane and labdane diterpenoids, strychnine, and denatonium, J Agric Food Chem, 55 (2007) 6236-6243.
[15] V. Rangbulla, A. Nirola, M. Gupta, P. Batra, M. Gupta, Salivary IgA, Interleukin-1β and MMP-8 as Salivary Biomarkers in Chronic Periodontitis Patients, Chinese Journal of Dental Research, 20 (2017) 43-51.
[16] H.M. Hernandez-Rios P, Garrido M, Tervahartiala T, Leppilahti J, Kuula H, Heikkinen AM, Mäntylä P, Rathnayake N, Nwhator S, Sorsa T. , Oral fluid matrix metalloproteinase (MMP)-8 as a diagnostic tool in chronic periodontitis, Metalloproteinases In Medicine., 2016;3:11-18.
[17] O. Schilling, C.M. Overall, Proteome-derived, database-searchable peptide libraries for identifying protease cleavage sites, Nature Biotechnology, 26 (2008) 685-694.
[18] I. Luchian, A. Goriuc, D. Sandu, M. Covasa, The Role of Matrix Metalloproteinases (MMP-8, MMP-9, MMP-13) in Periodontal and Peri-Implant Pathological Processes, International Journal of Molecular Sciences, 23 (2022).
[19] M.R. Guimaraes-Stabili, M.C. de Medeiros, D. Rossi, A.C. Camilli, C.F. Zanelli, S.R. Valentini, L.C. Spolidorio, K.L. Kirkwood, C. Rossa, Silencing matrix metalloproteinase-13 (Mmp-13) reduces inflammatory bone resorption associated with LPS-induced periodontal disease in vivo, Clinical Oral Investigations, 25 (2021) 3161-3172.
[20] I. Luchian, M. Moscalu, A. Goriuc, L. Nucci, M. Tatarciuc, I. Martu, M. Covasa, Using Salivary MMP-9 to Successfully Quantify Periodontal Inflammation during Orthodontic Treatment, Journal of Clinical Medicine, 10 (2021).
[21] W.T.Y. Loo, M. Wang, L.J. Jin, M.N.B. Cheung, G.R. Li, Association of matrix metalloproteinase (MMP-1, MMP-3 and MMP-9) and cyclooxygenase-2 gene polymorphisms and their proteins with chronic periodontitis, Archives of Oral Biology, 56 (2011) 1081-1090.
[22] L. Meinel, Aminomethyl-functionalized denatonium derivatives, their preperation and use, in, 2021.
[23] M. Schnabelrauch, Chewing gum for the diagnosis of inflammatory tissues in dental applications, in, 2014.
[24] H. Nagase, G.B. Fields, Human matrix metalloproteinase specificity studies using collagen sequence-based synthetic peptides, Peptide Science, 40 (1996) 399-416.
[25] U. Eckhard, P.F. Huesgen, O. Schilling, C.L. Bellac, G.S. Butler, J.H. Cox, A. Dufour, V. Goebeler, R. Kappelhoff, U. auf dem Keller, T. Klein, P.F. Lange, G. Marino, C.J. Morrison, A. Prudova, D. Rodriguez, A.E. Starr, Y. Wang, C.M. Overall, Active site specificity profiling datasets of matrix metalloproteinases (MMPs) 1, 2, 3, 7, 8, 9, 12, 13 and 14, Data in Brief, 7 (2016) 299-310.
[26] O. Schilling, U. auf dem Keller, C.M. Overall, Factor Xa subsite mapping by proteome-derived peptide libraries improved using WebPICS, a resource for proteomic identification of cleavage sites, Biological chemistry, 392 (2011) 1031-1037.
[27] L. Zhang, X. Li, H. Yan, L. Huang, Salivary matrix metalloproteinase (MMP)-8 as a biomarker for periodontitis: A PRISMA-compliant systematic review and meta-analysis, Medicine (Baltimore), 97 (2018) e9642-e9642.
[28] C.M. Cobb, Non-surgical pocket therapy: mechanical, Ann Periodontol, 1 (1996) 443-490.
[29] A.M. Marcaccini, C.A. Meschiari, L.R. Zuardi, T.S. De Sousa, M. Taba Jr, J.M. Teofilo, A.L.B. Jacob-Ferreira, J.E. Tanus-Santos, A.B. Novaes Jr, R.F. Gerlach, Gingival crevicular fluid levels of MMP-8, MMP-9, TIMP-2, and MPO decrease after periodontal therapy, Journal of Clinical Periodontology, 37 (2010) 180-190.
[30] G. Matuliene, B.E. Pjetursson, G.E. Salvi, K. Schmidlin, U. Brägger, M. Zwahlen, N.P. Lang, Influence of residual pockets on progression of periodontitis and tooth loss: Results after 11 years of maintenance, Journal of Clinical Periodontology, 35 (2008) 685-695.
[31] C.-C. Wang, H.-C. Houng, C.-L. Chen, P.-J. Wang, C.-F. Kuo, Y.-S. Lin, J.-J. Wu, M.T. Lin, C.-C. Liu, W. Huang, W.-J. Chuang, Solution Structure and Backbone Dynamics of Streptopain, Journal of Biological Chemistry, 284 (2009) 10957-10967.
[32] G.M. Air, Influenza neuraminidase, Influenza Other Respir Viruses, 6 (2012) 245-256.
[33] D.E. Bassi, H. Mahloogi, L. Al-Saleem, R. Lopez De Cicco, J.A. Ridge, A.J.P. Klein-Szanto, Elevated furin expression in aggressive human head and neck tumors and tumor cell lines, Molecular Carcinogenesis, 31 (2001) 224-232.
[34] O. Schilling, U. auf dem Keller, C.M. Overall, Protease Specificity Profiling by Tandem Mass Spectrometry Using Proteome-Derived Peptide Libraries, in: K. Gevaert, J. Vandekerckhove (Eds.) Gel-Free Proteomics: Methods and Protocols, Humana Press, Totowa, NJ, 2011, pp. 257-272.
[35] W. Nesse, F. Abbas, I. Van Der Ploeg, F.K.L. Spijkervet, P.U. Dijkstra, A. Vissink, Periodontal inflamed surface area: quantifying inflammatory burden, Journal of Clinical Periodontology, 35 (2008) 668-673.
[36] J. Geiger, Both, S., Kircher, S., Neumann, M., Rosenwald, A. and Jahns, R., Hospital-integrated Biobanking as a Service - The Interdisciplinary Bank of Biomaterials and Data Wuerzburg (ibdw). Open Journal of Bioresources, 5, p.6. (2018).

## Claims

1. A compound having the structure Den-R-NH-Pep, or a salt thereof, wherein Den is an optionally substituted denatonium residue,
R is an optionally substituted C₁₋₃ alkylene group, particularly wherein R is CH₂,
Pep is a protease-sensitive peptide,
wherein the C-terminus of said peptide forms an amide bond with the group -NH-,
and wherein said protease-sensitive peptide Pep comprises amino acid residues having the sequence P4-P3-P2-P1-P1'-P2'-P3'-P4', wherein
P4 is G,
P3 is independently selected from A and P,
P2 is independently selected from L and Q,
P1 is independently selected from G, A and E, preferably from A and E,
P1' is independently selected from I, L and Y,
P2' is independently selected from V and W,
P3' is G, and
P4' is A.

2. The compound or salt of claim 1, having the formula [I]: 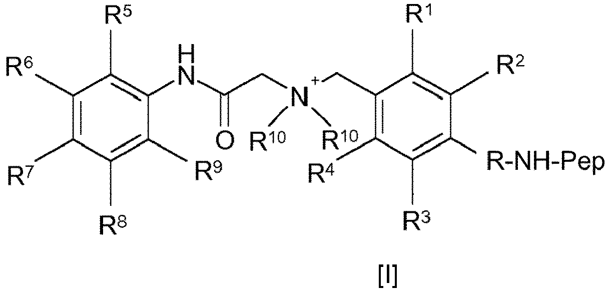
wherein
R¹⁰ is hydrogen or C₁-C₁₀-alkyl,
R¹-R⁹ independently are hydrogen, halogen, C₁-C₅-alkyl, C₁-C₄-alkoxy, C₁-C₂₀-alkoxycarbonyl,
R and Pep are as defined in claim 1;
particularly wherein
R¹⁰, R⁵ and R⁹ is C₁-C₄-alkyl,
R¹-R⁴ and R⁶-R⁸ is hydrogen,
R and Pep are as defined in claim 1.

3. The compound or salt of claim 1 or 2, respectively, wherein the compound of claim 1 has the formula [Ia]: 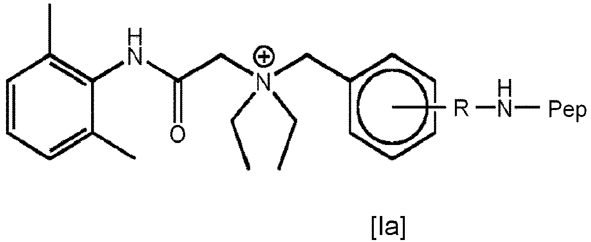
and wherein R and Pep are as defined in claim 1;
or wherein the compound of claim 2 has the formula [Ib]: 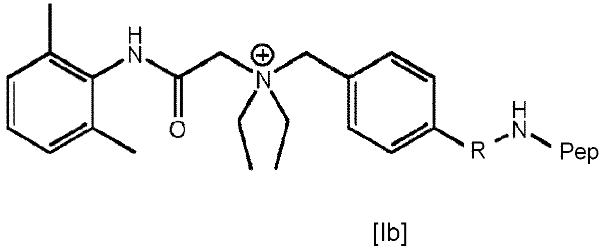
and wherein R and Pep are as defined in claim 1

4. The compound or salt of any one of claims 1 to 3, wherein the protease-sensitive peptide comprises at least 8 amino acids, preferably comprises 8 to 24 amino acids, more preferably comprises 8 to 16 amino acids, more preferably consists of 8 to 16 amino acids.

5. The compound or salt of any one of claims 1 to 4,
wherein said protease-sensitive peptide Pep comprises amino acid residues having the sequence P4-P3-P2-P1-P1'-P2'-P3'-P4', wherein
P4 is G,
P3 is independently selected from A and P,
P2 is L,
P1 is independently selected from A and E,
P1' is independently selected from I and L,
P2' is V,
P3' is G, and
P4' is A.
or wherein said protease-sensitive peptide Pep comprises amino acid residues having a sequence selected from GPQAYWGA (SEQ ID NO: 10) and GPLAYWGA (SEQ ID NO: 11).

6. The compound or salt of any one of claims 1 to 5,
wherein said protease-sensitive peptide Pep comprises amino acid residues having a sequence selected from the group consisting of GPLELVGA (SEQ ID NO: 2), GPLEIVGA (SEQ ID NO: 3), GPLALVGA (SEQ ID NO: 4), GPLAIVGA (SEQ ID NO: 5), GALELVGA (SEQ ID NO: 6), GALEIVGA (SEQ ID NO: 7), GALALVGA (SEQ ID NO: 8), and GALAIVGA (SEQ ID NO: 9), GPQAYWGA (SEQ ID NO: 10) and GPLAYWGA (SEQ ID NO: 11),
particularly wherein said protease-sensitive peptide Pep comprises amino acid residues having a sequence selected from the group consisting of GPLELVGA (SEQ ID NO: 2), GPLEIVGA (SEQ ID NO: 3), GPLALVGA (SEQ ID NO: 4), GPLAIVGA (SEQ ID NO: 5), GALALVGA (SEQ ID NO: 8) and GPQAYWGA (SEQ ID NO: 10).

7. The compound or salt of any one of claims 1 to 6, wherein said protease-sensitive peptide Pep further comprises one or two linker sequences, particularly wherein each of said linker sequences comprises at least 4 amino acids, preferably 4 to 15 amino acids,
especially wherein said protease-sensitive peptide Pep further comprises two linker sequences L1 and L2, which are preferably positioned on the N- and C-terminal side, respectively, of the amino acid residues defined in any of claims 5 and 6.

8. The compound or salt of any one of claims 1 to 7, wherein the protease-sensitive peptide Pep comprises a stabilizing N-terminal modification,
particularly wherein the protease-sensitive peptide Pep is acetylated at its N-terminus.

9. The compound or salt of any one of the preceding claims, wherein the protease is a pathogen-specific protease,
particularly wherein the said protease is a matrix metalloproteinase, especially wherein the said protease is selected from the group consisting of MM-1, MM-8, and MM-9,
especially wherein the protease is MM-8.

10. The compound or salt according to any one of claims 1 to 9, comprising a counter ion selected from the group consisting of F⁻, Cl⁻, Br⁻, 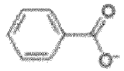 and

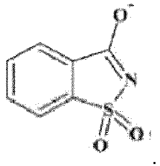

11. A bioresponsive sensor comprising the compound or salt of any one of claims 1 to 10.

12. The compound or salt according to any one of claims 1 to 10, or the bioresponsive sensor according to claim 11, for use in medicine, particularly for use in a method of detecting a medical condition, especially an inflammation in the oral cavity of a human patient.

13. A method for the preparation of the bioresponsive sensor, comprising the step of linking an amino-modified denatonium compound to the C-terminus of a protease-sensitive peptide,
wherein the amino-modified denatonium compound is preferably a compound of formula [IIa] or formula [IIb] 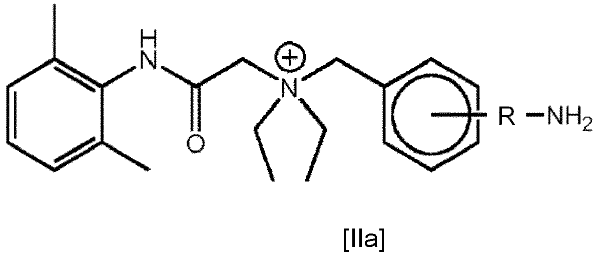 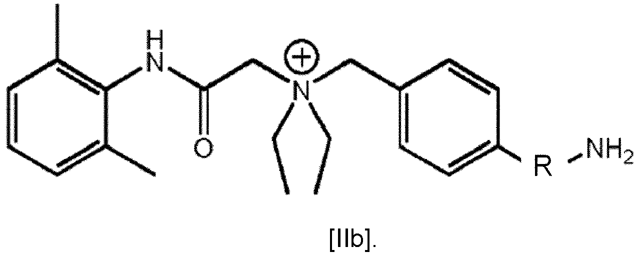
in particular wherein the method further comprises, prior to the step of linking:
- preparing a protease-sensitive peptide by solid phase peptide synthesis, and/or
- protecting the N-terminus of the protease-sensitive peptide with a protecting group, preferably acetic anhydride.

14. A diagnostic chewing gum or a diagnostic confectionary, comprising the compound or salt of any one of claims 1 to 10.

15. A protease-sensitive peptide having a length of from 8 to 16 amino acids,
wherein said protease-sensitive peptide Pep comprises amino acid residues having the sequence P4-P3-P2-P1-P1'-P2'-P3'-P4', wherein
P4 is G, P3 is independently selected from A and P, P2 is independently selected from L and Q, P1 is independently selected from G, A and E, preferably from A and E, P1' is independently selected from I, L and Y, P2' is independently selected from V and W, P3' is G, and P4' is A,
particularly wherein said peptide is further characterized as in any of claims 1 to 12.
